# EUROPEAN PATENT SPECIFICATION

(11) **EP 3 497 448 B1**
(45) Date of publication and mention of the grant of the patent: **15.11.2023**
(21) Application number: 17838228.9
(22) Date of filing: 11.08.2017
(51) Int. Cl.: G01N 33/574, G01N 33/564

(54) **IMMUNE STATUS BIOMARKERS AND USES THEREFOR**
IMMUNSTATUSBIOMARKER UND VERWENDUNGEN DAFÜR
BIOMARQUEURS DE L'ÉTAT IMMUNITAIRE ET UTILISATIONS DE CES DERNIERS

(30) Priority: 11.08.2016 AU 2016903160
(43) Date of publication of application: 19.06.2019
(73) Proprietor: The Council of the Queensland Institute of Medical Research, Herston, Queensland 4006 (AU)
(72) Inventor: WYKES, Michelle, Toowong Queensland 4066 (AU)
(74) Representative: J A Kemp LLP
(86) International application number: PCT/AU2017/050855
(87) International publication number: WO 2018/027281

(56) References cited:
- WO-A1-2016/008005
- WO-A1-2018/027252
- WANG PAN-LIANG ET AL: "Expression of programmed death-1 and its ligands in the liver of biliary atresia", WORLD JOURNAL OF PEDIATRICS, HANGZHOU INSITUTE OF PEDIATRICS : SPRINGER, CN, vol. 13, no. 6, 22 March 2017 (2017-03-22) , pages 604-610, XP036344802, ISSN: 1708-8569, DOI: 10.1007/S12519-017-0018-5 [retrieved on 2017-03-22]
- SPENCER?C. LIANG ET AL: "Regulation of PD-1, PD-L1, and PD-L2 expression during normal and autoimmune responses", EUROPEAN JOURNAL OF IMMUNOLOGY, vol. 33, no. 10, 1 October 2003 (2003-10-01), pages 2706-2716, XP055466758, Weinheim ISSN: 0014-2980, DOI: 10.1002/eji.200324228
- P'ng Loke ET AL: "PD-L1 and PD-L2 Are Differentially Regulated by Th1 and Th2 Cells", Proceedings of the National Academy of Sciences of the United States of America, 29 April 2003 (2003-04-29), pages 5336-5341, XP055670953, United States DOI: 10.1073/pnas.0931259100 Retrieved from the Internet: URL:https://www.pnas.org/content/pnas/100/ 9/5336.full.pdf [retrieved on 2020-03-23]
- MASAYOSHI ISHIDA ET AL: "Differential expression of PD-L1 and PD-L2, ligands for an inhibitory receptor PD-1, in the cells of lymphohematopoietic tissues", IMMUNOLOGY LETTERS, vol. 84, no. 1, 1 October 2002 (2002-10-01), pages 57-62, XP055203928, ISSN: 0165-2478, DOI: 10.1016/S0165-2478(02)00142-6
- BAPTISTA MAURICIO Z ET AL: "Prognostic significance of PD-L1 and PD-L2 in breast cancer", HUMAN PATHOLOGY, SAUNDERS, PHILADELPHIA, PA, US, vol. 47, no. 1, 25 September 2015 (2015-09-25), pages 78-84, XP029329871, ISSN: 0046-8177, DOI: 10.1016/J.HUMPATH.2015.09.006
- KARUNARATHNE DESHAPRIYA S ET AL: "Programmed Death-1 Ligand 2-Mediated Regulation of the PD-L1 to PD-1 Axis Is Essential for Establishing CD4+T Cell Immunity", IMMUNITY, CELL PRESS, US, vol. 45, no. 2, 16 August 2016 (2016-08-16), pages 333-345, XP029687766, ISSN: 1074-7613, DOI: 10.1016/J.IMMUNI.2016.07.017
- MATAKI, N. ET AL.: 'Expression of PD-1, PD-L1, and PD-L2 in the Liver in Autoimmune Liver Diseases' AMERICAN JOURNAL OF GASTROENTEROLOGY vol. 102, 2007, pages 302 - 312, XP055463429
- JUNG, H. I. ET AL.: 'Overexpression of PD-L1 and PD-L2 Is Associated with Poor Prognosis in Patients with Hepatocellular Carcinoma' CANCER RESEARCH AND TREATMENT vol. 49, no. 1, 2017, pages 246 - 254, XP055463433
- OHIGASHI, Y. ET AL.: 'Clinical Significance of Programmed Death-1 Ligand-1 and Programmed Death-1 Ligand-2 Expression in Human Esophageal Cancer' CLINICAL CANCER RESEARCH vol. 11, no. 8, 2005, pages 2947 - 2953, XP002419631

## Description

### FIELD OF THE INVENTION

This application claims priority to Australian Provisional Application No. 2016903160 entitled "Immunostimulatory Compositions and Uses Therefor" filed 11 August 2016.

This invention relates generally to compositions, methods, for diagnosing and/or monitoring the Th1 immune status of a subject. The invention can be used for diagnosis, monitoring, making treatment decisions, or management of subjects suspected of having Th1-related disease. More particularly, the present invention relates to peripheral blood biomarkers that are useful for determining the Th1 immune status of the subject.

### BACKGROUND OF THE INVENTION

Programmed cell death protein 1 (PD-1) is recognized as an important player in immune regulation, through its actions as a brake on effector T cells and in reducing immune responses in the tissue microenvironment. PD-1 is expressed on activated T cells including immunosuppressive CD4⁺ T cells (Treg) and exhausted CD8⁺ T cells, but also on B cells, myeloid dendritic cells (MDCs), monocytes, thymocytes, and natural killer (NK) cells. This broad PD-1 expression suggests a wide implication of the PD-1 signaling pathway needed for effective immunity and maintenance of T cell homeostasis (Gianchecchi et al., Autoimmun. Rev. 12: 1091-100, 2013).

Significantly, the PD-1 signaling pathway contributes to the maintenance of both central and peripheral tolerance in normal individuals. In the thymus, the interaction of PD-1 and its ligands suppresses positive selection thereby inhibiting the transformation of CD4⁻ CD8⁻ double negative cells to CD4⁺ CD8⁺ double positive T cells (Keir et al., J. Immunol. 175:7329-7379, 2005). PD-1 signaling is also responsible for inhibition of self-reactive and inflammatory effector T cells that escape negative selection to avoid collateral immune-mediated tissue damage (Keir et al., J. Exp. Med. 203:883-895, 2006).

PD-1 has two known ligands: protein death ligand 1 (PD-L1; Freeman et al., J. Exp. Med. 192:1027-34, 2000), also known as B7-H1 in humans (Dong et al., Nat. Med. 5:1365-9, 1999), and protein death ligand 2 (PD-L2; Latchman et al., Nat. Immunol. 2:261-8, 2001), also known as B7-DC (Tseng et al., J. Exp. Med. 193:839-46, 2001). The patterns of expression of PD-L1 and PD-L2 are quite distinct. PD-L1 is expressed constitutively by a wide variety of immune cells and non-immune cells and appears to be upregulated in most normal tissue cells in the presence of strong inflammatory signals (Matzinger et al., Nat. Rev. Immunol. 11:221-230, 2011; Mühlbauer et al., J. Hepatol. 45:520-528, 2006; Pinchuk et al., Gastroenterol. 135:1228-1237, 2008; Stanciu et al, J. Infect. Dis., 193:404-412, 2006). By contrast, constitutive basal expression of PD-L2 is low compared to PD-L1, and although PD-L2 expression was initially thought to be restricted to antigen-presenting cells (APCs) such as monocytes, macrophages and dendritic cells (DCs) (Latchman et al., Nature Immunol. 2:261-268, 2001; Yamazaki et al., J. Immunol. 169:5538-5545, 2002), several groups have recently shown that PD-L2 expression can be induced on a wide variety of other immune cells and non-immune cells depending on microenvironmental stimuli (Kinter et al., J. Immunol. 181:6738-6746, 2008; Zhong et al., Eur. J. Immunol. 37:2405-2410, 2007; Messal et al., Mol. Immunol. 48:2214-2219, 2011; Lesterhuis et al., Mol. Immunol. 49:1-3, 2011).

PD-1 and its ligands are aberrantly expressed by malignant cells and surrounding microenvironmental cells. Within the tumor microenvironment, PD-1 is highly expressed on a large proportion of tumor-infiltrating lymphocytes (TILs) from many different tumor types and suppresses local effector immune responses. TIL expression of PD-1 is associated with impaired effector function (cytokine production and cytotoxic efficacy against tumor cells) and/or poor outcome in several tumor types (Thompson et al., Clin. Cancer Res. 13:1757-1761, 2007; Zhang et al., Mol. Immunol. 7:389-395, 2010; Ahmadzadeh M et al., Blood 114: 1537-1544, 2009; Shi et al., Int. J. Cancer 128:887-896, 2011), including renal cell carcinoma, metastatic melanoma, as well as stomach, breast, ovarian, pancreatic, and lung cancers. Similarly, PD-L2 has been observed to be upregulated in a subset of human tumors and has occasionally been linked to poor outcome (Rozali et al., Clin. Dev. Immunol. 2012:656340, 2012).

Given its potential role in cancer-associated immune suppression in the tumor microenvironment, targeting the PD-1/PD-ligand pathway has been proposed as an attractive treatment strategy. Several studies in this regard have investigated the therapeutic effect of blocking antibodies against the PD-1/PD-L1 pathway, demonstrating enhanced tumor control rates, (Curran et al., Proc. Natl. Acad Sci. USA 107:4275-4280, 2010; Iwai et al., Proc. Natl. Acad Sci. USA 99:12293-12297, 2002; Pilon-Thomas et al., J. Immunol. 184:3442-3449, 2010; Zhang et al., Blood 114:1545-1552). However, few studies have investigated blocking of PD-L2 as a defined treatment strategy. Although in a few studies PD-L2 blocking strategies were used, this was always in combination with the targeting of PD-L1 (Parekh et al., J. Immunol. 182:2816-1826, 2009; He et al., J. Immunol. 173:4919-4928, 2004), which did not permit deducing the true value of anti-PD-L2 strategies.

### SUMMARY OF THE INVENTION

The present invention is predicated in part on the determination that PD-L2 expression on cells such as APCs, which interact with immune system effector cells, inversely correlates with the severity of Th1-related diseases and that PD-L2 is required to establish Th1 immunity. Therefore, the present inventors determined that PD-L2 is a reliable indicator of an upregulated and/or enhanced Th1 immune response in a subject. The present inventors have also discovered other biomarkers that are modulated during a Th1 immune response. Inclusion of these additional biomarkers increases the diagnostic power and reliability of the diagnostic and prognostic assays taught herein. Based on these determinations, it is proposed that PD-L2, optionally in combination with other Th1 immune status biomarkers, is indicative of the Th1 immune status of a subject, and has utility for tracking Th1 immune status development in subjects suffering from Th1-related diseases.

Accordingly, in one aspect, the present invention provides methods for determining an indicator used in assessing a subject's Tₕ1 immune status, the method comprising: (1) determining a Th1 immune status biomarker profile of a sample obtained from the subject, wherein the Th1 immune status biomarker profile comprises a biomarker value for at least one Th1 immune status biomarker in the sample, wherein the at least one Th1 immune status biomarker comprises programmed cell death protein 1 ligand 2 (PD-L2) of immune effector cell (IEC)-interacting cells in the sample; and (2) determining the indicator using the biomarker value(s), wherein the indicator is at least partially indicative of the Th1 immune status of the subject.

Suitably, the IEC-interacting cells are selected from APCs and tumor cells. In a related aspect, the present invention provides methods for determining an indicator for use in assessing the Th1 immune status of a subject, the method comprising: (1) determining a Th1 immune status biomarker profile of a sample obtained from the subject, wherein the Th1 immune status biomarker profile comprises a biomarker value for at least one Th1 immune status biomarker in the sample, wherein the at least one Th1 immune status biomarker comprises PD-L2, and optionally PD-L1, of APCs in the sample; and (2) determining the indicator using the biomarker value(s), wherein the indicator is at least partially indicative of the Th1 immune status of the subject. The APC is suitably selected from the group consisting of a dendritic cell or a macrophage. In representative examples of this type, the APC is a CD11c-expressing dendritic cells.

Suitably, in any of the aspects or embodiments disclosed herein, the biomarker value(s) is(are) at least partially indicative of a concentration of the Th1 immune status biomarker in the sample obtained from the subject, and in some embodiments of this type the biomarker value includes the abundance of the Th1 immune status biomarker. In a representative example, an individual biomarker value includes the percentage of IEC-interacting cells (e.g., APCs or tumor cells) that express the Th1 immune status biomarker on the cell surface (e.g., PD-L2⁺ dendritic cells). In some embodiments of this type, the Th1 immune status biomarker is PD-L2 and the biomarker value is a measurement of PD-L2 clustering on the surface of an IEC-interacting cell (e.g., an APC, such as a dendritic cell).

When determining the Th1 immune status of a subject, in some embodiments the level of PD-L2 is reduced in the sample relative to a control level of PD-L2 that correlates with the presence of normal or unimpaired Th1 immunity, and the indicator is thereby determined to be at least partially indicative of impaired Th1 immunity. In other embodiments, the level of PD-L2 in the sample is about the same as a control level of PD-L2 that correlates with the presence of normal or unimpaired Th1 immunity, and the indicator is therefore determined to be at least partially indicative of normal or unimpaired Th1 immunity. In still other embodiments, the level of PD-L2 in the sample is increased relative to a control level of PD-L2 that correlates with the presence of normal or unimpaired Th1 immunity, and the indicator is therefore determined to be at least partially indicative of elevated Th1 immunity.

The indicator used in assessing Th1 immune status is made more reliable and of greater diagnostic power when the at least one Th1 immune status biomarkers further comprises PD-L1. Accordingly, in some embodiments the biomarker values from a pair of Th1 immune status biomarkers are used to determine the indicator. For example, in some preferred embodiments, the pair of biomarkers are PD-L2 and PD-L1. When more than one Th1 immune status biomarker is used in the methods of the invention, the method suitably further comprises applying a combining function to the biomarker values. In this regard, illustrative examples of suitable combining functions are selected from the group comprising: an additive model; a linear model; a support vector machine; a neural network model; a random forest model; a regression model; a genetic algorithm; an annealing algorithm; a weighted sum; a nearest neighbor model; and a probabilistic model.

Preferably, the methods described above and elsewhere herein comprise: (a) determining a biomarker value for a first Th1 immune status biomarker; (b) determining a corresponding biomarker value for a second Th1 immune status biomarker; (c) determining the indicator using the biomarker values recorded on the first and second Th1 immune status biomarkers, the indicator being indicative of a ratio of the biomarker values recorded on the first and second Th1 immune status biomarkers. In illustrative methods of this type, the first Th1 immune status biomarker is PD-L2, and the second Th1 immune status biomarker is PD-L1. By way of an example, in some embodiments the ratio of the first and second Th1 immune status biomarker values determined from the sample ("the sample Th1 immune status biomarker ratio") is reduced relative to a control PD-L2:PD-L1 biomarker value ratio that correlates with the presence of normal or unimpaired Th1 immunity, and the indicator is determined to be at least partially indicative of impaired Th1 immunity. For example, the sample biomarker value ratio is suitably no more than about 95%, 94%, 93%, 92%, 91%, 90%, 80%, 70%, 60%, 50%, 40%, 30%, 20%, or 10% (and every integer in between) of the control biomarker value ratio of (e.g., determined from a control sample obtained from a subject with a normal or unimpaired Th1 immune response).

Conversely, when the sample PD-L2:PD-L1 biomarker value ratio is increased relative to a control PD-L2:PD-L1 biomarker value ratio that correlates with the presence of normal or unimpaired Th1 immunity, the indicator is determined to be at least partially indicative of elevated Th1 immunity. In these instances, the sample PD-L2:PD-L1 biomarker value ratio is at least about 105%, 106%, 107%, 108%, 109%, 110%, 120%, 130%, 140% 150%, 160%, 170%, 180%, 190%, 200%, 250%, 300%, 400%, 500%, 600%, 700%, 800%, 900%, or 1000% (and every integer in between) of the control biomarker value ratio (e.g., determined from a control sample obtained from a subject with a normal or unimpaired Th1 immune response).

In other embodiments, when the sample PD-L2:PD-L1 biomarker value ratio is about the same as a control PD-L2:PD-L1 biomarker value ratio that correlates with the presence of normal or unimpaired Th1 immunity, the indicator is determined to be at least partially indicative of normal or unimpaired Th1 immunity. In these instances, the sample PD-L2:PD-L1 biomarker value ratio is usually from about 96% to 104% (and all integer percentages in between) of the control biomarker value ratio (*e*.*g*., determined from a control sample obtained from a subject with a normal or unimpaired Th1 immune response).

Any known techniques for measuring protein biomarkers or nucleic acid biomarkers are suitable for use with the present invention. For example, the biomarkers can be measured using flow cytometry, immunoassays, mass spectrometry, sequencing platforms, array and hybridization platforms, or a combination thereof.

Suitable immunoassays include enzyme-linked immunosorbent assays (ELISA) or a radioimmunoassay (RIA). In some preferred embodiments, the biomarker value is measured using flow cytometry.

The inventors' findings enable methods of diagnosing diseases and/or conditions with an undesirable Th1 immune status (also referred to interchangeably herein as "Th1-related diseases" or "Th1-related disorders"), as well as for monitoring treatment regimens that are prescribed for treating said diseases. Accordingly, in another aspect, the present invention provides a method as described above and elsewhere herein, wherein the indicator is used to diagnose the presence or absence of a disease. In some embodiments, the disease is associated with a reduced or suppressed Th1 immune status, and is diagnosed when the level of PD-L2 in the sample obtained from the subject is below a predetermined threshold. For example, the disease that is associated with a reduced or suppressed Th1 immune status could be a metastatic cancer or a pathogenic infection.

In other embodiments, the indicator can be used to diagnose a Th1-related disease that is associated with an elevated Th1 immune response, and is therefore diagnosed when the level of PD-L2 in the sample obtained from the subject is above a predetermined threshold. Exemplary diseases of this type include autoimmune diseases. For example, the autoimmune disease can be selected from any one of the group comprising: ankylosing spondylitis, Barrett's esophagus, chronic fatigue syndrome (CFS/CFIDS/ME), chronic Lyme disease (borreliosis), Crohn's disease, diabetes, depression, fibromyalgia (FM), gastroesophageal reflux disease (GERD), Hashimoto's thyroiditis, hypertension, hyperthyroidism, hypothyroidism, irritable bowel syndrome (IBS), interstitial cystitis (IC), kidney stones, Löfgren's syndrome, systemic lupus erythematosus (SLE), multiple chemical sensitivity (MCS), migraine headache, Morgellon's, multiple sclerosis, osteoarthritis, polymyalgia rheumatic, prostatitis, psoriasis, psoriatic arthritis, Raynaud's syndrome/phenomenon, reactive arthritis (Reiter syndrome), restless leg syndrome, reflex sympathetic dystrophy (RSD), rheumatoid arthritis, sarcoidosis, scleroderma, sinusitis, seasonal affective disorder (SAD), Sjögren's syndrome, ulcerative colitis, uveitis, and vertigo.

In still another aspect, the present invention provides a method for determining an indicator that is useful for distinguishing between a metastatic cancer and a non-metastatic cancer in a subject, the method comprising: (1) determining a Th1 immune status biomarker profile of a sample obtained from the subject, wherein the Th1 immune status biomarker profile comprises a biomarker value for at least one (*i*.*e*., 1, 2, 3, 4, 5, 6, 7, 8, 9, 10, or more then 10) Th1 immune status biomarker in the sample, wherein the at least one Th1 immune status biomarker comprises PD-L2 of IEC-interacting cells (e.g., APCs or tumor cells) in a sample; and (2) determining the indicator using the biomarker value(s), wherein the indicator is at least partially capable of distinguishing between metastatic cancer and non-metastatic cancer in a subject. Suitably, the at least one biomarker value is at least partially indicative of a concentration of PD-L2 in the sample obtained from the subject. In some embodiments of this type, the IEC-interacting cells (*e*.*g*., APCs such as dendritic cells), and the at least one biomarker value is at least partially indicative of the percentage of dendritic cells that express PD-L2⁺ on the cell surface. As such, the cancer can be determined likely to be non-metastatic when the PD-L2 biomarker value indicates PD-L2 expression on at least around 60% of the dendritic cells in the sample obtained from the subject. Conversely, the cancer can be determined likely to be metastatic when the PD-L2 biomarker value indicates PD-L2 expression on less than about 50% of the dendritic cells of the sample.

In some embodiments, the Th1 immune status biomarker profile comprises a biomarker value for PD-L1 of the IEC-interacting cells (*e*.*g*., APCs, such as dendritic cells). As described above, in some embodiments the biomarker values of PD-L2 and PD-L1 are at least partially indicative of the percentage of IEC-interacting cells (*e*.*g*., APCs, such as dendritic cells) expressing these biomarkers on the cell surface. In some embodiments of this type the indicator is indicative of a ratio of the percentages of the IEC-interacting cells (*e*.*g*., APCs, such as dendritic cells) in the sample that express PD-L2 and PD-L1 biomarkers.

In some embodiments of this type the IEC-interacting cells are dendritic cells. For example, a PD-L2:PD-L1 biomarker value ratio of between around 0.9 to 1.3 is indicative that the subject has a normal or unimpaired Th1 immunity and is therefore likely to be suffering from a non-metastatic cancer (*e*.*g*., a benign lesion). Alternatively, a PD-L2:PD-L1 biomarker value ratio of between around 0.4 to 0.8 is indicative that the subject has a reduced Th1 immunity and is likely to be suffering from a metastatic cancer.

Yet another aspect provides compositions for determining an indicator used in assessing a subject's Tₕ1 immune status, or for diagnosing diseases and/or conditions with an undesirable Th1 immune status, or for monitoring disease progression or regression, or for determining an indicator that is useful for distinguishing between a metastatic cancer and a non-metastatic cancer in a subject, the compositions comprising, consisting or consisting essentially of IEC-interacting cells (e.g., APCs or tumor cells), a PD-L2 detection agent and optionally a PD-L1 detection agent. In some embodiments, the IEC-interacting cells have a PD-L2:PD-L1 ratio of between about 0.9 to 1.3. In other embodiments, the IEC-interacting cells have a PD-L2:PD-L1 ratio of between about 0.4 to 0.8. Suitably, the PD-L2 detection agent and/or PD-L1 detection agent are bound to PD-L2 and/or PD-L1, respectively, on the surface of the IEC-interacting cells. The surface PD-L2 and/or PD-L1 may be in the form of clusters. The PD-L2 and/or PD-L1 detection agents are suitably antibodies. In specific embodiments, the PD-L2 and/or PD-L1 detection agents comprise a covalently attached label.

### BRIEF DESCRIPTION OF THE DRAWINGS

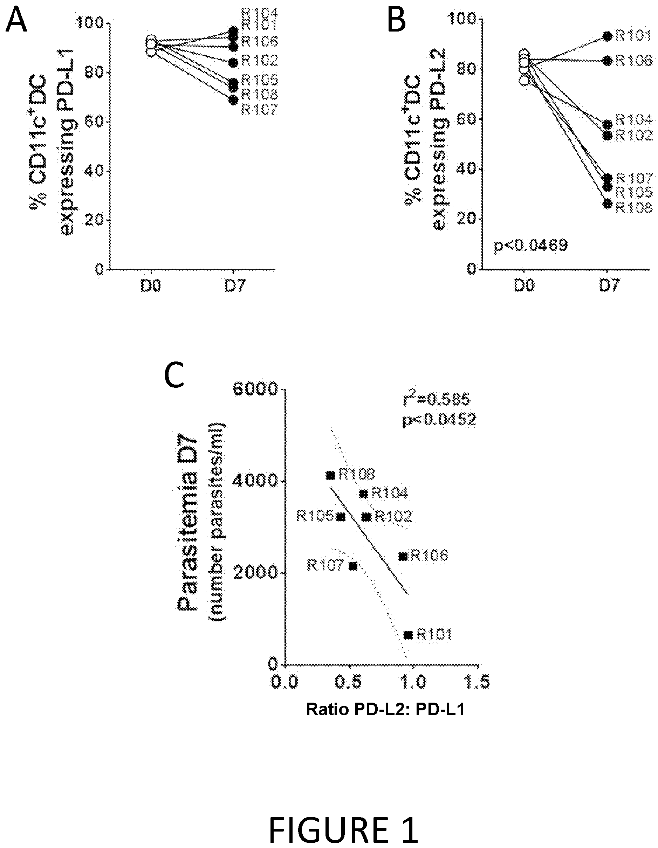

Figure 1 is a graphical representation output from the FACS analysis characterizing biomarker expression on cell surface. PD-L2 expression on DCs inversely correlates with malaria parasitemia in humans. (A-C) Seven healthy human volunteers were inoculated with *P. falciparum* and blood examined for percentage of CD11c⁺ DC expressing (A) PD-L1 and (B) PD-L2, before and seven days after infection. (C) Plot showing number of parasites per mL of blood versus ratio of %PD-L2: %PD-L1 DC. R101 to R108 represents each volunteer. The p value is testing the null hypothesis that the overall slope is zero.

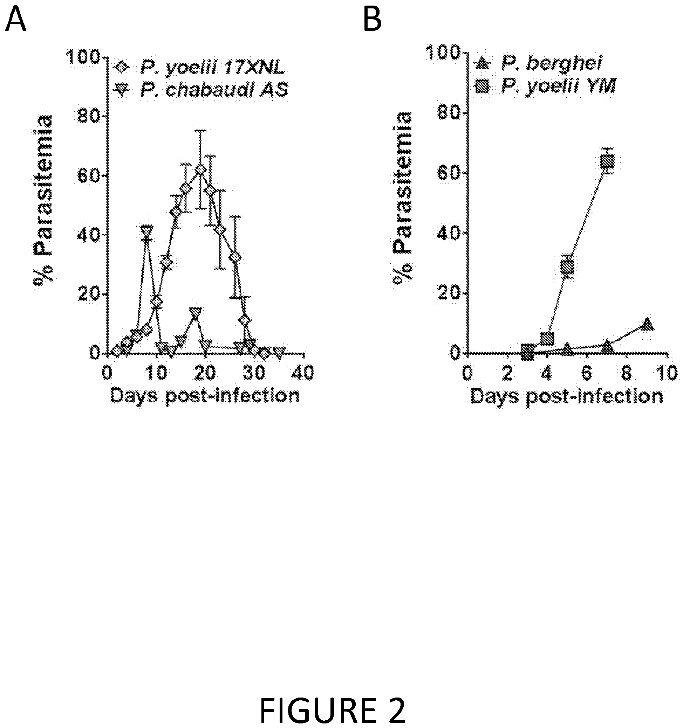

Figure 2 is a graphical representation showing (A) mean percent parasitemia for typical courses of infection in mice infected with non-lethal *P. chabaudi* or *P. yoelii* 17XNL malaria and monitored for up to 40 days. (B) Mean percent parasitemia for typical courses of infection in mice infected with lethal *P. yoelii* YM or *P. berghei* and monitored for 10 days. Error bars represent SEM (n = 4-8).

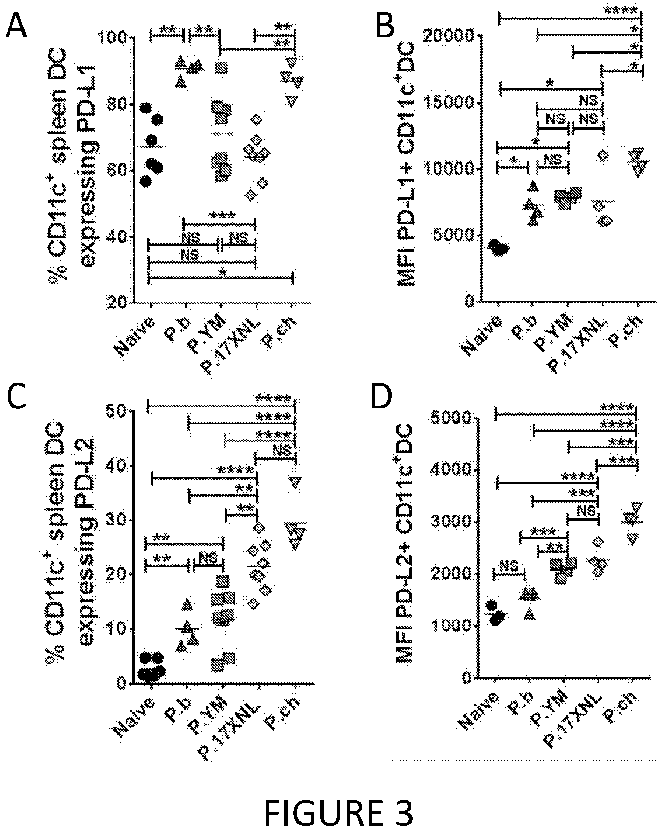

Figure 3 is a graphical representation showing (A) the percentage of total CD11c⁺ DC expressing PD-L1 and (B) Mean Fluorescence Intensity (MFI) of surface PD-L1-expression on PD-L1⁺ CD11c⁺ spleen DCs from naive and infected mice (seven days post infection). (C) Percentage of total CD11c⁺ DC expressing PD-L2 and (D) MFI of surface PD-L2-expression on PD-L2⁺ CD11c⁺ spleen DCs from native and infected mice (day 7 post infection). Bars on scatter plots represent mean value. Significance between matched day 0 and day 7 human samples was analyzed by Wilcoxon matched-pairs signed rank test. Significance between multiple groups was analyzed using one way ANOVA with Tukey's multiple comparisons test. (* p < 0.05; ** p < 0.01; *** p < 0.001; **** p < 0.0001 are for comparisons between groups). Data for (A) and (C) represent pooled independent experiments in which similar results were obtained.

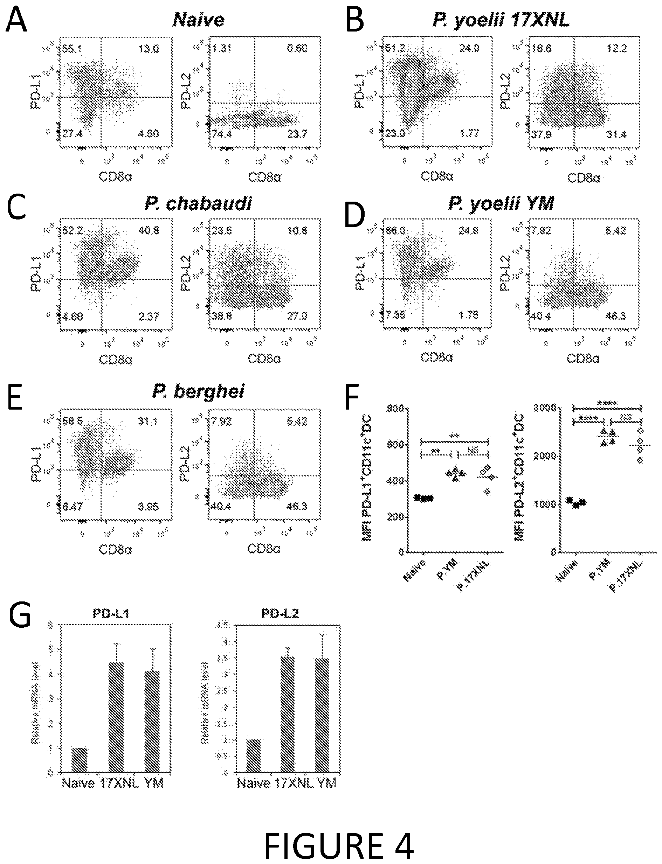

Figure 4 is a graphical representation showing PD-L1 and PD-L2 expression levels on DCs from lethal and non-lethal malaria. (A)-(E) Flow cytometric profiles of PD-L1, PD-L2 and CD8 expression on viable CD19⁻ CD3⁻ D11c⁺ DCs from (A) naïve mice and mice infected with malaria strains (B) non-lethal *P. yoelii* 17XNL or (C) non-lethal *P. chabaudi* (D) lethal *P. yoelii* YM or (E) lethal *P. berghei.* (F) Repeat experiment for MFI of CD11c⁺ spleen DCs from naive and infected mice (seven days post infection) expressing PD-L1 and PD-L2, measured on a different flow cytometer with different voltage settings to that used in Figures 3B and D. (G) Quantitative RT-PCR analysis of PD-L1 and PD-L2 from RNA isolated from DCs (naive or infected with *P. yoelii* 17XNL and YM at day seven). Results are normalised to the geometric mean of three housekeeping genes (CxxC1, TBP and mRPL13A). Values are expressed as mean ± SEM of three independent experiments, and expressed relative to results from uninfected mice.

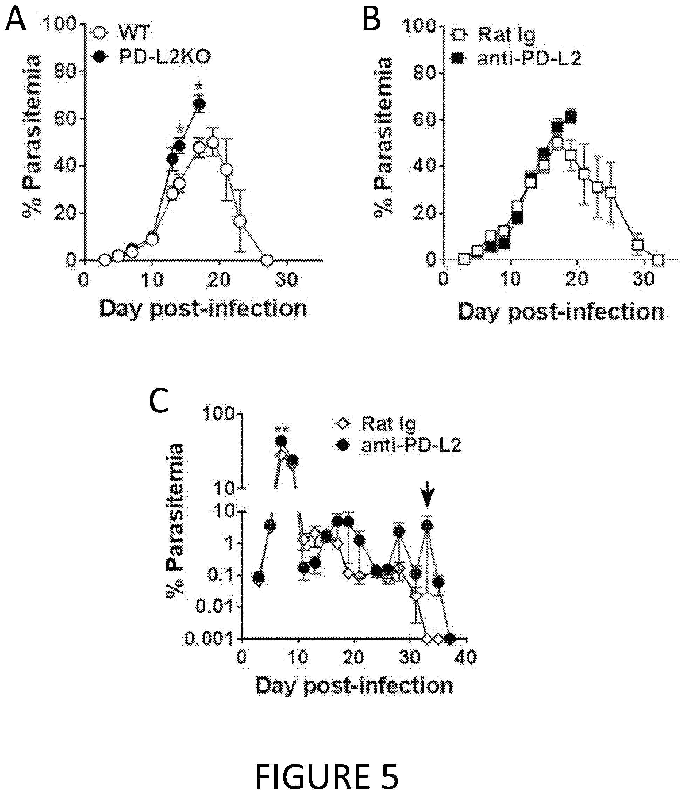

Figure 5 is a graphical representation showing that PD-L2 improves immunity and survival from malaria infection. (A-C) mean percent parasitemia for a typical course of *P. yoelii* 17XNL malaria in (A) PD-L2 ko and wild-type mice (n = 4) or (B) wild-type mice treated with Rat IgG or anti-PD-L2 blocking antibody (n = 5); and (C) mean percent parasitemia (on a log scale) for a typical course of *P. chabaudi* malaria in wild-type mice treated with Rat IgG or anti-PD-L2 blocking antibody (n = 5). Arrow indicates parasites were cleared four days earlier in rat IgG than anti-PD-L2-treated mice. Data represent one of two independent experiments that obtained similar results. Significance at certain time points were analyzed using the non-parametric Mann-Whitney *U* test based on 2-sided tail. Error bars represent SEM (* p < 0.05; ** p < 0.005).

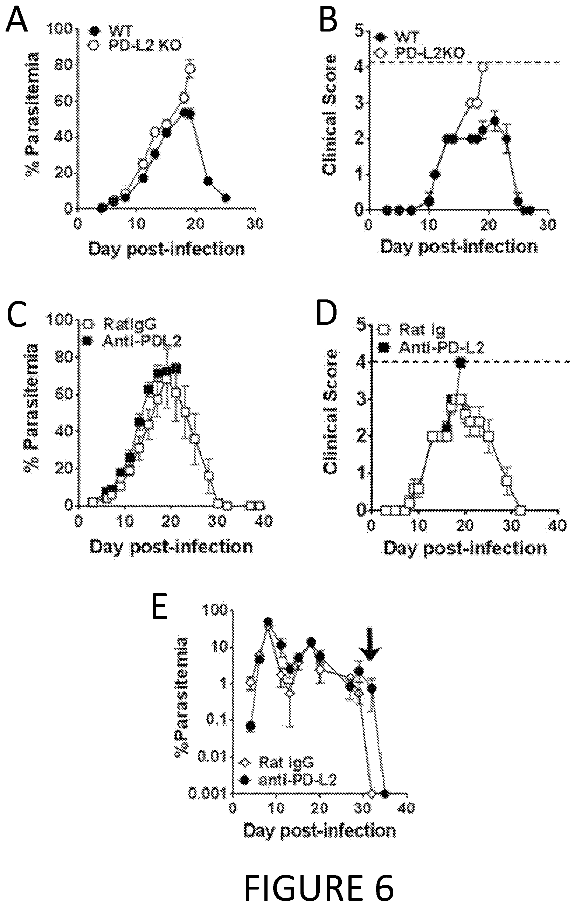

Figure 6 is a graphical representation showing that PD-L2 regulates protection, symptoms and Th1 immunity during *P. yoelii* 17XNL malaria. (A-D) Parasitemia and clinical symptom scores from duplicate experiments for Figures 5A and B, during a typical course of P. *yoelii* 17XNL malaria in (A)-(B) wild-type mice and PD-L2 ko mice (n = 5) or (C)-(D) wild-type mice treated with rat IgG or anti-PD-L2 blocking antibody (n = 5). (E) Parasitemia during typical course of *P*. *chabaudi* malaria in wild-type mice treated with Rat IgG or anti-PD-L2 blocking antibody (n = 5) in a duplicate experiment as described for Figure 2C. Arrow indicates parasites were cleared three days earlier in Rat IgG than anti-PD-L2-treated mice.

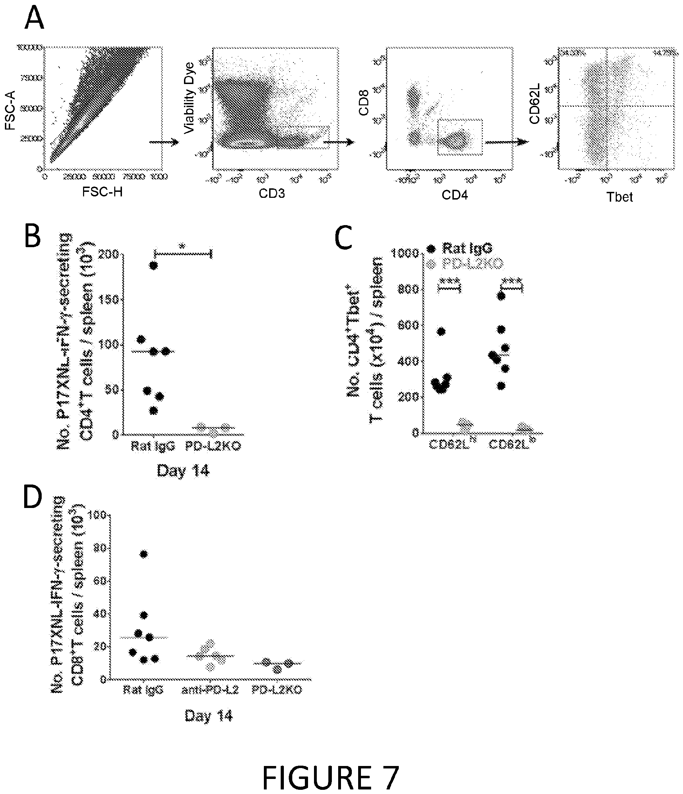

Figure 7 is a graphical representation showing (A) the gating strategy used to assess T_{bet} expression in CD4⁺ T cells by flow cytometry. (B)-(C) Scatter plots show number of T cells per spleen, in wild-type mice treated with rat IgG (n = 7) or anti-PD-L2 blocking antibody (n = 7) or in PD-L2 ko mice (n = 3) infected with *P. yoelii* 17XNL for 14 days. (B) Mean numbers of T_{bet}-expressing CD4⁺ CD62L^{hi} or CD4⁺ CD62L^{lo} T cells per spleen. (C) Mean numbers of CD4⁺ T cells per spleen, that secreted IFN-γ in an ELISPOT culture in response to parasite antigen (MSP1₁₉), in the presence of naive DCs. (D) Scatter plot shows number of CD8⁺ T cells per spleen, at day 14, that secreted IFN-γ in an ELISPOT culture in response to parasite peptide (Pb1), in the presence of naive DCs. The data are pooled from 2 independent experiments except for PD-L2 ko mice which were assessed once. Significance was analyzed using the non-parametric Mann-Whitney U test based on 2-sided tail. (* p < 0.05; *** p < 0.001).

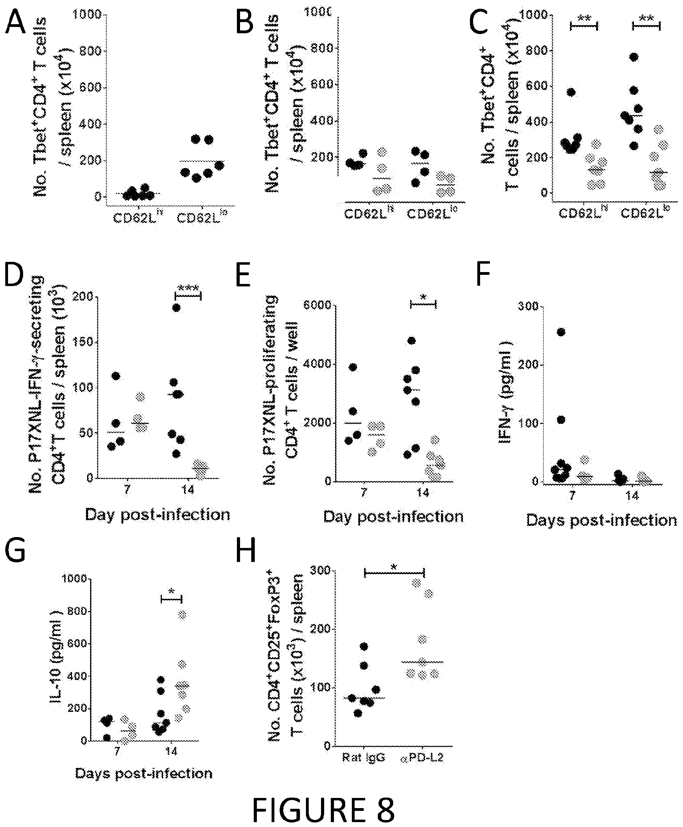

Figure 8 is a graphical representation showing that blockade of PD-L2 inhibits the expansion of parasite-specific CD4⁺ T cells in mice infected with *P. yoelii* 17XNL. Wild-type mice infected with *P. yoelii* 17XNL and treated with rat IgG or anti-PD-L2 blocking antibody (n = 7). (A), (B), (C) Numbers of T_{bet}-expressing CD4⁺ CD62L^{hi} and CD4⁺ CD62L^{lo} T cells per spleen on (A) day 0; (B) day 7; and (C) day 14; (D) Numbers of CD4⁺ T cells that secreted Interferon-γ (IFN-γ) in an ELISPOT culture in response to parasite antigen (MSP1₁₉) in the presence of naive DCs. (E) Numbers of CD4⁺ T cells that proliferated in cultures in response to parasite antigen MSP1₁₉ in the presence of naive DCs, measured by incorporation of EdU; (F) and (G) Mean levels of (F) IFN-γ; and (G) IL-10 in the serum *P. yoelii* 17XNL-infected mice. (H) Mean numbers of CD4⁺ T cells expressing CD25 and FoxP3 (regulatory T cells) per spleen. Bar on scatter plots represent median values. The data represent two pooled independent experiments. Significance was analyzed using the non-parametric Mann-Whitney U test based on two-sided tail (* p < 0.05; ** p < 0.01; *** p < 0.001).

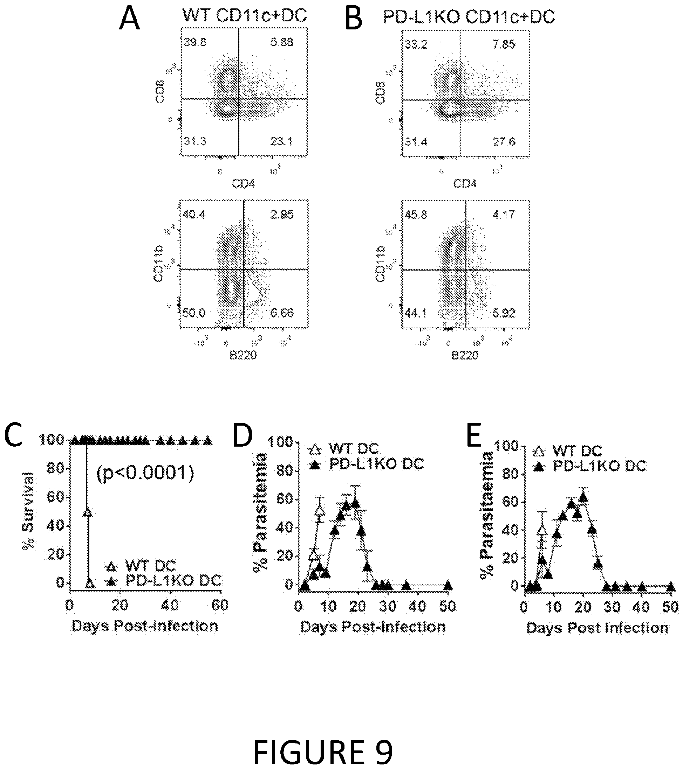

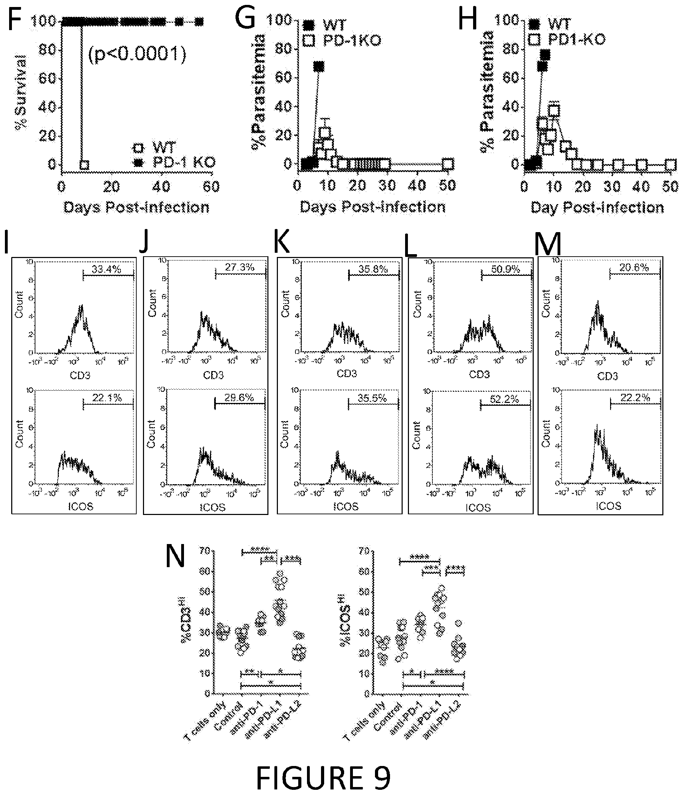

Figure 9 is a graphical representation showing the differential effect of DC-expressed PD-L1 and PD-L2 on T cells and immunity. Flow cytometry profiles of CD19⁻ CD3⁻CD11c⁺ DC from (A) wild-type; and (B) PD-L1 ko mice, taken at day 7 of a lethal *P. yoelii* YM infection and labeled for markers of DC sub-populations (CD4⁺; CD8⁺; B220⁺ pDC; and CD11b⁺ DC). (C) Survival curves showing protection against lethal malaria by DCs without PD-L1 expression. Wile-type and PD-L1 ko mice were infected with lethal dose of 10⁴ *P. yoelii* YM pRBC , DCs were isolated from infected (drug-cured) mice and 1 × 10⁷ DCs were transferred to each mouse in groups of four naive mice, in duplicate experiments. After 24 hours, each mouse was infected with 10⁴ *P. yoelii* YM pRBC, and survival monitored every 1-3 days for 50 days. (D)-(E) Duplicate experiments in which parasitemia in naïve mice transfused with ~ 1 × 10⁷ DC from wild-type and PD-L1 ko mice, taken at day 7 of a lethal *P. yoelii* YM infection. After 24 hours, each transfused mouse was infected with 10⁴ *P. yoelii* YM pRBC and monitored every 1-3 days for 50 days. Results are mean ± SEM, n = 4 mice/ group. (F) Survival curves showing PD-1 ko mice are immune to lethal malaria. Groups of five wild-type and PD-1 ko mice were infected with lethal 10⁴ *P. yoelii* YM pRBC and survival monitored every 1-3 days for 50 days in duplicate experiments. (G)-(H) Duplicate experiments in which parasitemia in wild-type and PD-1 ko mice infected with 10⁴ *P. yoelii* YM pRBC and monitored every 1-3 days for 50 days. Results are mean ± SEM, n = 5 mice/ group. (I)-(M) Flow cytometry analysis of CD3 and ICOS expression on CD4⁺ CD62L^{lo} PD-1⁺ T cells cultured with DCs expressing PD-L1 and PD-L2; wherein (I) is a negative control comprising only T cells without DCs; (J) is a positive control comprising T cells and DCs; (K) blockade of PD-1; (L) blockade of PD-L1; and (G) blockade of PD-L2; after 36 hours. Both T cells and DCs were isolated from the spleens of mice infected with *P. yoelii* 17XNL for 12-14 days. Gates to determine high CD3 or ICOS expression were chosen based on clear double peaks found in anti-PD-L1 cultures. (N) Scatter plots showing percentages of CD4⁺ CD62^{lo} T cells/well with high CD3 and ICOS expression in replicate wells (n = 3 - 5) from three independent experiments shown as white, pale blue and darker blue spots. Error bars represent mean. Significance was analyzed using the unpaired t-test (one-sided tail) from one of the three experiments (* p < 0.05; ** p < 0.005; *** p < 0.0005; **** p < 0.0001).

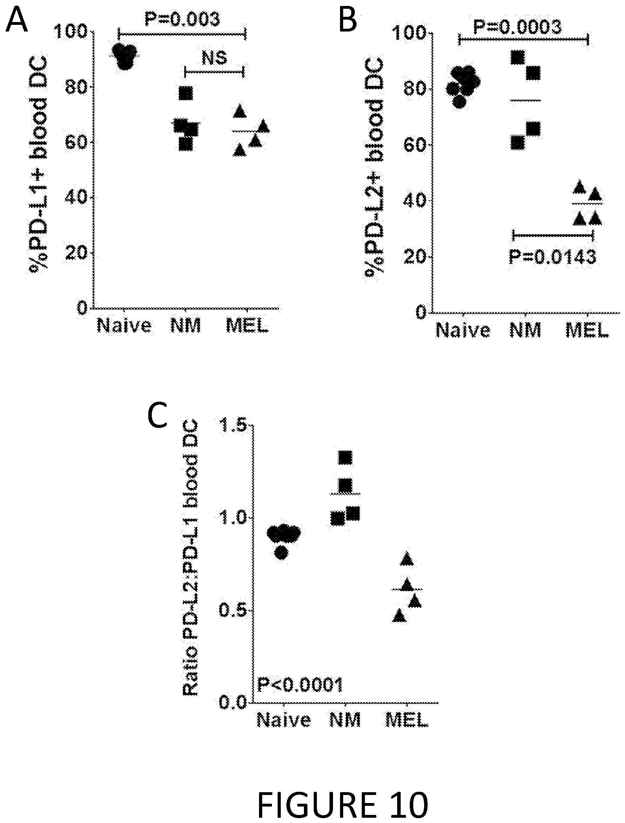

Figure 10 is a graphical representation showing that PD-L2 expression on blood DCs is reduced in patients with metastatic melanomas. (A-C) Blood was taken from eight healthy human volunteers, four patients with non-melanoma lesions and four patients with metastatic melanomas. Their blood was examined for percentage of CD11c⁺ DC expressing (A) PD-L1 and (B) PD-L2. (C) Plot showing ratio of %PD-L2: %PD-L1 DCs in each group. The P value for (A) and (B) used Mann Whitney test between each group and (C) was calculated by Kruskal-Wallis multiple comparison test.

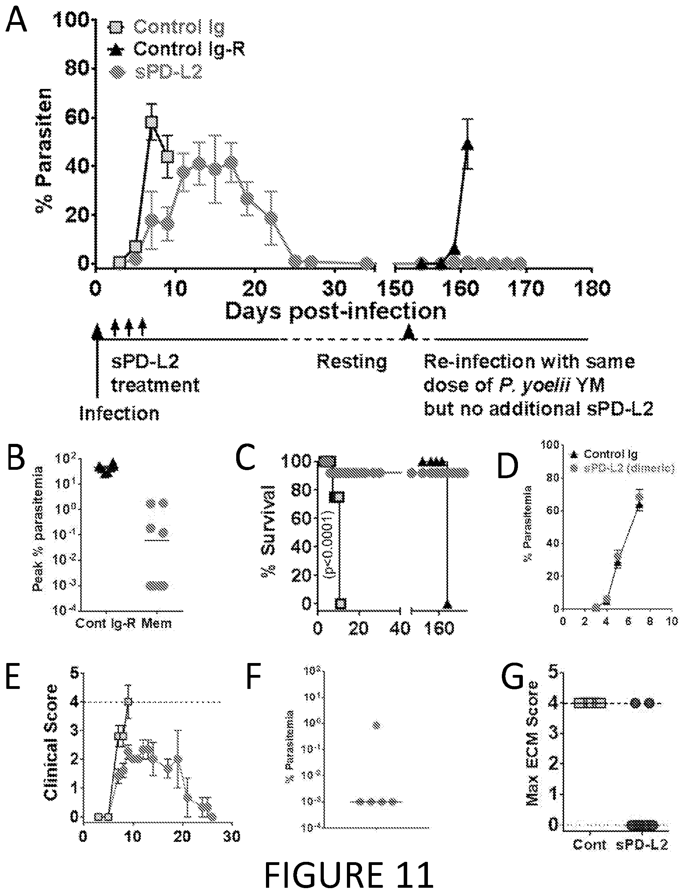

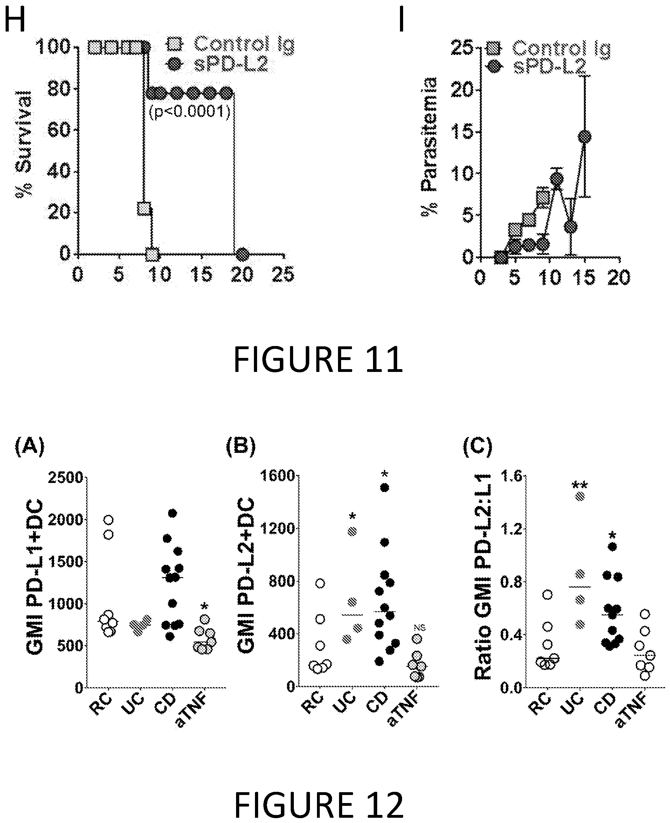

Figure 11 is a graphical representation showing that multimerized PD-L2 protects against lethal malaria. (A) Mean percent parasitemia; (B) log-scale showing number of mice with parasitemia; (C) survival (x-axis showing number days post infection); (D) Percentage parasitemia for a typical course of *P. yoelii* YM malaria in wild-type mice treated with negative control (human) IgG or dimeric PD-L2 on days 3, 5 and 7 post infection. and (E) clinical symptom scores (x-axis showing number days post infection) for a typical course of *P. yoelii* YM malaria in wild-type mice treated with control (human) IgG or PD-L2 after detectable parasitemia, on day 3 and then days 5 and 7 (total n = 12, from three independent experiments). All surviving mice were rested and after 150 days, rechallenged with the same dose of lethal *P. yoelii* YM malaria (no additional PD-L2 was administered) along with new age-matched control mice (control Ig-R). (F) Peak percent parasitemia in naïve mice given 200 µl blood from PD-L2-treated mice, 20 days after rechallenge with *P. yoelii* YM (x-axis showing number days post infection). This assay detects low numbers of parasite in the blood of donor mice. (G) Clinical symptom scores, (H) survival (x-axis showing number days post infection), and (I) mean percent parasitemia (x-axis showing number days post infection) for a typical course of *P. berghei* infection in wild-type mice treated with control (human) IgG or PD-L2 on days 3, 5 and 7 post-infection (total n = 9 from two independent experiments). Error bars represent SEM. Significance of survival was analyzed using Log-rank (Mantel-Cox) test.
Figure 12 is a graphical representation showing that PD-L2 expression on blood DCs is increased in patients with IBD but not after TNF blockade (aTNF). (A-B) Blood was taken from 7 healthy human volunteers, 12 Crohns Disease (CD) and 4 ulcerative colitis patients (UC) and 7 after treatment with TNF blockade. Their blood was examined for Geometric Mean Fluorescence intensity (GMI) of CD11c⁺ DC expressing (a) PD-L1 and (b) PD-L2. (c) Plot showing ratio of GMI PD-L2: GMI PD-L1 DCs in each group. The P value used unpaired t test on samples. Bars = Median. *p<0.05; **p<0.01

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one *(i.e.,* to at least one) of the grammatical object of the article. By way of example, "an element" means one element or more than one element.

As used herein the terms "abundance," "level" and "amount" are used interchangeably herein to refer to a quantitative amount (e.g., weight or moles), a semiquantitative amount, a relative amount (e.g., weight % or mole % within class), a concentration, and the like. Thus, these terms encompass absolute or relative amounts or concentrations of Th1 immune status biomarkers in a sample.

As used herein, "and/or" refers to and encompasses any and all possible combinations of one or more of the associated listed items, as well as the lack of combinations when interpreted in the alternative (or).

The term "antibody" and its grammatical equivalents refer to a protein which is capable of specifically binding to a target antigen such as PD-L2 or any other surface molecule on an APC and includes any substance, or group of substances, which has a specific binding affinity for an antigen, suitably to the exclusion of other substances. This term encompasses an immunoglobulin molecule capable of specifically binding to a target antigen by virtue of an antigen binding site contained within at least one variable region. This term includes four chain antibodies (e.g., two light chains and two heavy chains), recombinant or modified antibodies (e.g., chimeric antibodies, humanized antibodies, primatized antibodies, de-immunized antibodies, half antibodies, bispecific antibodies) and single domain antibodies such as domain antibodies and heavy chain only antibodies (e.g., camelid antibodies or cartilaginous fish immunoglobulin new antigen receptors (IgNARs)). An antibody generally comprises constant domains, which can be arranged into a constant region or constant fragment or fragment crystallizable (Fc). In specific embodiments, the antibodies comprise a four-chain structure as their basic unit. Full-length antibodies comprise two heavy chains (≈50-70 kDa) covalently linked and two light chains (≈23 kDa each). A light chain generally comprises a variable region and a constant domain and in mammals is either a K light chain or a λ light chain. A heavy chain generally comprises a variable region and one or two constant domain(s) linked by a hinge region to additional constant domain(s). Heavy chains of mammals are of one of the following types α, δ, ε, γ, or µ. Each light chain is also covalently linked to one of the heavy chains. For example, the two heavy chains and the heavy and light chains are held together by inter-chain disulfide bonds and by non-covalent interactions. The number of inter-chain disulfide bonds can vary among different types of antibodies. Each chain has an N-terminal variable region (V_{H} or V_{L} wherein each are ≈110 amino acids in length) and one or more constant domains at the C-terminus. The constant domain of the light chain (C_{L} which is ≈110 amino acids in length) is aligned with and disulfide bonded to the first constant domain of the heavy chain (C_{H} which is ≈330-440 amino acids in length). The light chain variable region is aligned with the variable region of the heavy chain. The antibody heavy chain can comprise two or more additional C_{H} domains (such as, C_{H2}, C_{H3} and the like) and can comprise a hinge region can be identified between the C_{H1} and Cm constant domains. Antibodies can be of any type (e.g., IgG, IgE, IgM, IgD, IgA, and IgY), class (e.g., IgG₁, IgG₂, IgG₃, IgG₄, IgA₁ and IgA₂) or subclass. In one example, the antibody is a murine (mouse or rat) antibody or a primate (suitably human) antibody. The term "antibody" encompasses not only intact polyclonal or monoclonal antibodies, but also variants, fusion proteins comprising an antibody portion with an antigen binding site, humanized antibodies, human antibodies, chimeric antibodies, primatized antibodies, de-immunized antibodies or veneered antibodies. Also within the scope of the term "antibody" are antigen binding fragments that retain specific binding affinity for an antigen, suitably to the exclusion of other substances. This term includes a Fab fragment, a Fab' fragment, a F(ab') fragment, a single chain antibody (SCA or SCAB) amongst others. An "Fab fragment" consists of a monovalent antigen-binding fragment of an antibody molecule, and can be produced by digestion of a whole antibody molecule with the enzyme papain, to yield a fragment consisting of an intact light chain and a portion of a heavy chain. An "Fab' fragment" of an antibody molecule can be obtained by treating a whole antibody molecule with pepsin, followed by reduction, to yield a molecule consisting of an intact light chain and a portion of a heavy chain. Two Fab' fragments are obtained per antibody molecule treated in this manner. An "F(ab')2 fragment" of an antibody consists of a dimer of two Fab' fragments held together by two disulfide bonds, and is obtained by treating a whole antibody molecule with the enzyme pepsin, without subsequent reduction. A (Fab')₂ fragment. An "Fv fragment" is a genetically engineered fragment containing the variable region of a light chain and the variable region of a heavy chain expressed as two chains. A "single chain antibody" (SCA) is a genetically engineered single chain molecule containing the variable region of a light chain and the variable region of a heavy chain, linked by a suitable, flexible polypeptide linker.

The term "antigen-presenting cells" (APCs) refers to a class of cells capable of presenting one or more antigens in the form of peptide-MHC complex recognizable by specific effector cells of the immune system (also referred to herein as "immune effector cells" or "IECs"), and thereby modulating (e.g., stimulating/enhancing or reducing/tolerizing/anergizing) an immune response to the antigen or antigens being presented. In specific embodiments of the present invention, the APCs are capable of activating IECs such as T lymphocytes, including CD8⁺ and/or CD4⁺ lymphocytes. Cells that have in vivo the potential to act as APC include, for example, not only professional APCs such as dendritic cells, macrophages, Langerhans cell, monocytes and B cells but also non-professional APCs illustrative examples of which include activated epithelial cells, fibroblasts, glial cells, pancreatic beta cells and vascular endothelial cells. Many types of cells are capable of presenting antigens on their cell surface for IEC, including T cell, recognition.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

The term "biomarker" typically refers to a measurable characteristic that reflects the presence or nature (e.g., severity or status) of a physiological and/or pathophysiological state, including an indicator of risk of developing a particular physiological or pathophysiological state. For example, a biomarker may be present in a sample obtained from a subject before the onset of a physiological or pathophysiological state, including a symptom, thereof. Thus, the presence of the biomarker in a sample obtained from the subject is likely to be indicative of an increased risk that the subject will develop the physiological or pathophysiological state or symptom thereof. Alternatively, or in addition, the biomarker may be normally expressed in an individual, but its expression may change (i.e., it is increased (upregulated; over-expressed) or decreased (downregulated; under-expressed) before the onset of a physiological or pathophysiological state, including a symptom thereof. Thus, a change in the level of the biomarker is likely to be indicative of an increased risk that the subject will develop the physiological or pathophysiological state or symptom thereof. Alternatively, or in addition, a change in the level of a biomarker may reflect a change in a particular physiological or pathophysiological state, or symptom thereof, in a subject, thereby allowing the nature (e.g., severity) of the physiological or pathophysiological state, or symptom thereof, to be tracked over a period of time. This approach may be useful in, for example, monitoring a treatment regimen for the purpose of assessing its effectiveness (or otherwise) in a subject. As herein described, reference to the level of a biomarker includes the concentration of a biomarker, or the level of expression of a biomarker, or the activity of the biomarker, as will be described in more detail below.

The term "biomarker value" refers to a value measured or derived for at least one corresponding biomarker of a subject and which is typically at least partially indicative of an abundance or concentration of a biomarker in a sample taken from the subject. Thus, the biomarker values could be measured biomarker values, which are values of biomarkers measured for the subject, or alternatively could be derived biomarker values, which are values that have been derived from one or more measured biomarker values, for example by applying a function to the one or more measured biomarker values. Biomarker values can be of any appropriate form depending on the manner in which the values are determined. For example, the biomarker values could be determined using high-throughput technologies such as mass spectrometry, sequencing platforms, array and hybridization platforms, immunoassays, flow cytometry, or any combination of such technologies. In one preferred example, the biomarker values relate to a level of activity or abundance of a protein expression product or other measurable molecule, quantified using a technique such as flow cytometry or the like. In this case, the biomarker values can be in the form of a percentage value of cells expressing the biomarker within a sample, as will be appreciated by persons skilled in the art and as will be described in more detail below.

The term "biomarker profile" refers to one or a plurality of one or more types of biomarkers (e.g., a polypeptide molecule, a cDNA molecule, etc.), or an indication thereof, together with a feature, such as a measurable aspect (e.g., biomarker value) of the biomarker(s). A biomarker profile may comprise a single biomarker whole level, abundance or amount correlates with the Th1 immune status of a subject (e.g., an enhanced Th1 immune status or a reduced Th1 immune status). Alternatively, a biomarker profile may comprise at least two such biomarkers or indications thereof, where the biomarkers can be in the same or different classes, such as, for example, a polypeptide and a nucleic acid. Thus, a biomarker profile may comprise at least 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, or 100 or more biomarkers or indications thereof. In some embodiments, a biomarker profile comprises a couple, several, tens, or hundreds of biomarkers or indications thereof. A biomarker profile can further comprise one or more controls or internal standards. In certain embodiments, the biomarker profile comprises at least one biomarker, or indication thereof, that serves as an internal standard. In other embodiments, a biomarker profile comprises an indication of one or more types of biomarkers. The term "indication" as used herein in this context merely refers to a situation where the biomarker profile contains symbols, data, abbreviations or other similar indicia for a biomarker, rather than the biomarker molecular entity itself. The term "biomarker profile" is also used herein to refer to a biomarker value or combination of at least two biomarker values, wherein individual biomarker values correspond to values of biomarkers that can be measured or derived from one or more subjects, which combination is characteristic of a Th1 immune status, discrete condition, stage of condition, subtype of condition or a prognosis for a discrete condition, stage of condition, subtype of condition. The term "profile biomarkers" is used to refer to a subset of the biomarkers that have been identified for use in a biomarker profile that can be used in performing a clinical assessment, such as to rule in or rule out a specific condition, different stages or severity of conditions, subtypes of different conditions or different prognoses. The number of profile biomarkers will vary, but is typically of the order of 10 or less.

By "clustering," and grammatical equivalents used herein, is meant any reversible or irreversible association of more than two of the same Th1 immune status biomarkers (e.g., PD-L2). Clusters can be made up of 3, 4, 5, 6, 7, 8, 9, 10, 12, 20, *etc.* biomarkers. Clusters of three or more biomarkers are generally termed oligomers, with individual numbers of clusters having their own designation, for example, a cluster of three biomarkers is a trimer, a cluster of four biomarkers is a tetramer, a cluster of five biomarkers is a pentamer, a cluster of six biomarkers is a hexamer, a cluster of seven biomarkers is a heptamer, a cluster of eight biomarkers is an octamer, a cluster of nine biomarkers is a nonamer, a cluster of ten biomarkers is a decamer, a cluster of twelve biomarkers is a dodecamer, and a cluster of twenty biomarkers is a eicosa mer.

Throughout this specification, unless the context requires otherwise, the words "comprise," "comprises" and "comprising" will be understood to imply the inclusion of a stated step or element or group of steps or elements but not the exclusion of any other step or element or group of steps or elements. Thus, use of the term "comprising" and the like indicates that the listed elements are required or mandatory, but that other elements are optional and may or may not be present. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The term "correlating" refers to determining a relationship between one type of data with another or with a state (e.g., Th1 immune status).

As used herein, the terms "diagnosis," "diagnosing" and the like are used interchangeably herein to encompass determining the likelihood that a subject will develop a condition, or the existence or nature of a condition in a subject. These terms also encompass determining the severity of disease or episode of disease, as well as in the context of rational therapy, in which the diagnosis guides therapy, including initial selection of therapy, modification of therapy (e.g., adjustment of dose or dosage regimen), and the like. By "likelihood" is meant a measure of whether a subject with particular measured or derived biomarker values actually has a condition (or not) based on a given mathematical model. An increased likelihood for example may be relative or absolute and may be expressed qualitatively or quantitatively. For instance, an increased likelihood may be determined simply by determining the subject's measured or derived biomarker values for at least two Th1 immune status biomarkers and placing the subject in an "increased likelihood" category, based upon previous population studies. The term "likelihood" is also used interchangeably herein with the term "probability". The term "risk" relates to the possibility or probability of a particular event occurring at some point in the future. "Risk stratification" refers to an arraying of known clinical risk factors to allow physicians to classify patients into a low, moderate, high or highest risk of developing a particular disease.

As used herein, the term "immune effector cells" (IECs) refers to a population of lymphocytes that display effector moiety receptors, e.g., cytokine receptors, and/or Fc receptors on their surface through which they bind an effector moiety, e.g., a cytokine, and/or an Fc region of an antibody and contribute to the destruction of target cells, e.g., tumor cells. IECs may for example mediate cytotoxic or phagocytic effects. IECs include, but are not limited to, effector T cells such as CD8⁺ cytotoxic T cells, CD4⁺ helper T cells, γδ T cells, NK cells, NK-like T cells, lymphokine-activated killer (LAK) cells, B cells and macrophages/monocytes.

The term "immune system", as used herein, refers to cells, molecular components and mechanisms, including antigen-specific and non-specific categories of the adaptive and innate immune systems, respectively, that provide a defense against damage and insults resulting from a viral infection. The term "innate immune system" refers to a host's non-specific reaction to insult to include antigen-nonspecific defense cells, molecular components and mechanisms that come into action immediately or within several hours after exposure to almost any insult or antigen. Elements of the innate immunity include for example phagocytic cells (monocytes, macrophages, dendritic cells, polymorphonuclear leukocytes such as neutrophils, reticuloendothelial cells such as Küpffer cells, and microglia), cells that release inflammatory mediators (basophils, mast cells and eosinophils), natural killer cells (NK cells) and physical barriers and molecules such as keratin, mucous, secretions, complement proteins, immunoglobulin M (IgM), acute phase proteins, fibrinogen and molecules of the clotting cascade, and cytokines. Effector compounds of the innate immune system include chemicals such as lysozymes, IgM, mucous and chemo-attractants (e.g., cytokines or histamine), complement and clotting proteins. The term "adaptive immune system" refers to antigen-specific cells, molecular components and mechanisms that emerge over several days, and react with and remove a specific antigen. The adaptive immune system develops throughout a host's lifetime. The adaptive immune system is based on leukocytes, and is divided into two major sections: the humoral immune system, which acts mainly *via* immunoglobulins produced by B cells, and the cell-mediated immune system, which functions mainly *via* T cells.

The term "indicator" as used herein refers to a result or representation of a result, including any information, number, ratio, signal, sign, mark, or note by which a skilled artisan can estimate and/or determine a likelihood or risk of whether or not a subject is suffering from a given disease. In the case of the present invention, the "indicator" may optionally be used together with other clinical characteristics, to arrive at a determination of the Th1 immune status of the subject. That such an indicator is "determined" is not meant to imply that the indicator is 100% accurate. The skilled clinician may use the indicator together with other clinical indicia to arrive at a diagnosis.

The term "nucleic acid" or "polynucleotide" as used herein includes RNA, mRNA, miRNA, cRNA, cDNA mtDNA, or DNA. The term typically refers to a polymeric form of nucleotides of at least 10 bases in length, either ribonucleotides or deoxynucleotides or a modified form of either type of nucleotide. The term includes single and double stranded forms of DNA or RNA.

By "obtained" is meant to come into possession. Samples so obtained include, for example, nucleic acid extracts or polypeptide extracts isolated or derived from a particular source. For instance, the extract may be isolated directly from a biological fluid or tissue of a subject.

The terms "PD-L2 expression" and "PD-L1 expression" refers to the transcription and/or translation and/or activity of PD-L2 and PD-L1 respectively. Several methods can be utilized to determine the level of PD-L2 and PD-L1 expression, as described in detail below.

As used herein, the term "positive response" means that the result of a treatment regimen includes some clinically significant benefit, such as the prevention, or reduction of severity, of symptoms, or a slowing of the progression of the condition. By contrast, the term "negative response" means that a treatment regimen provides no clinically significant benefit, such as the prevention, or reduction of severity, of symptoms, or increases the rate of progression of the condition.

"Protein," "polypeptide" and "peptide" are used interchangeably herein to refer to a polymer of amino acid residues and to variants and synthetic analogues of the same.

The term "prognosis" as used herein refers to a prediction of the probable course and outcome of a clinical condition or disease. A prognosis is usually made by evaluating factors or symptoms of a disease that are indicative of a favorable or unfavorable course or outcome of the disease (e.g., Th1 immune status). The skilled artisan will understand that the term "prognosis" refers to an increased probability that a certain course or outcome will occur; that is, that a course or outcome is more likely to occur in a subject exhibiting a given condition, when compared to those individuals not exhibiting the condition.

The term "sample" as used herein includes any biological specimen that may be extracted, untreated, treated, diluted or concentrated from a subject. Samples may include, without limitation, biological fluids such as whole blood, serum, red blood cells, white blood cells, plasma, saliva, urine, stool (i.e., faeces), tears, sweat, sebum, nipple aspirate, ductal lavage, tumor exudates, synovial fluid, ascitic fluid, peritoneal fluid, amniotic fluid, cerebrospinal fluid, lymph, fine needle aspirate, amniotic fluid, any other bodily fluid, cell lysates, cellular secretion products, inflammation fluid, semen and vaginal secretions. Samples may include tissue samples and biopsies, tissue homogenates and the like. Advantageous samples may include ones comprising any one or more biomarkers as taught herein in detectable quantities. Suitably, the sample is readily obtainable by minimally invasive methods, allowing the removal or isolation of the sample from the subject. In certain embodiments, the sample contains blood, especially peripheral blood, or a fraction or extract thereof. Typically, the sample comprises blood cells such as mature, immature or developing leukocytes, including lymphocytes, polymorphonuclear leukocytes, neutrophils, monocytes, reticulocytes, basophils, coelomocytes, hemocytes, eosinophils, megakaryocytes, macrophages, dendritic cells natural killer cells, or fraction of such cells (e.g., a nucleic acid or protein fraction). In specific embodiments, the sample comprises leukocytes including peripheral blood mononuclear cells (PBMC).

The terms "subject", "individual" and "patient" are used interchangeably herein to refer to an animal subject, particularly a vertebrate subject, and even more particularly a mammalian subject. Suitable vertebrate animals that fall within the scope of the invention include, but are not restricted to, any member of the phylum Chordata, subphylum vertebrata including primates, rodents (e.g., mice rats, guinea pigs), lagomorphs (e.g., rabbits, hares), bovines (e.g., cattle), ovines (e.g., sheep), caprines (e.g., goats), porcines (e.g., pigs), equines (e.g., horses), canines (e.g., dogs), felines (e.g., cats), avians (e.g., chickens, turkeys, ducks, geese, companion birds such as canaries, budgerigars etc.), marine mammals (e.g., dolphins, whales), reptiles (snakes, frogs, lizards, etc.), and fish. A preferred subject is a primate (e.g., a human, ape, monkey, chimpanzee). The subject suitably has at least one (e.g., 1, 2, 3, 4, 5 or more) clinical sign of a Th1immune status-associated disease.

A "Th1-related disease" or "Th1-related disorder" as used interchangeably herein refers to a disease that is associated with the development of a Th1 immune response. A "Th1 immune response" as used herein refers to the proliferation or increased differentiation of Th1 cells. A Th1-related disease or disorder is suitably identified by (1) levels of Th1 cells, Th1 cytokines and/or Th1 antibodies that exceed those normally found in a human, animal, or cell culture; (2) pathological findings associated with the disease or medical condition that can be mimicked experimentally in animals by administration of agents that upregulate proliferation or differentiation of Th1 cells; or (3) a pathology induced in experimental animal models of the disease or medical condition can be inhibited or abolished by treatment with agents that inhibit the proliferation or differentiation of Th1 cells. In most Th1-related diseases, at least two of the three conditions are met.

As used herein, the term "treatment regimen" refers to prophylactic and/or therapeutic (*i*.*e*., after onset of a specified condition) treatments, unless the context specifically indicates otherwise. The term "treatment regimen" encompasses natural substances and pharmaceutical agents (*i*.*e*., "drugs") as well as any other treatment regimen including but not limited to dietary treatments, physical therapy or exercise regimens, surgical interventions, and combinations thereof.

It will be appreciated that the terms used herein and associated definitions are used for the purpose of explanation only and are not intended to be limiting.

### 2. Th1 immune status biomarkers and their use

The present invention concerns methods, compositions, for identifying the Th1 immune status of a subject, or for providing a prognosis for subjects with a Th1-related disease. In particular, Th1 immune status biomarkers are disclosed for use in these modalities to determine the presence, absence or degree of a Th1 immune response in a subject, or for providing a prognosis for subjects with clinical symptoms of a Th1-related disease.

### 2.1 Th1 immune status biomarkers

The present inventors have determined that certain surface markers are present on cells that interact with IEC and that are specifically expressed in humans and mice during a Th1 immune response. The results presented herein provide clear evidence that a unique biologically-relevant biomarker profile predicts the Th1 immune status of a subject with a remarkable degree of accuracy. Overall, these findings provide compelling evidence that the IEC-interacting cell surface biomarkers disclosed herein, and particularly PD-L2, can function as biomarkers for determining Th1 immune status and may potentially serve as a useful diagnostic for triaging treatment decisions for subjects suffering with a disease associated with an undesirable Th1 immune status. In this regard, it is proposed that the methods, compositions, disclosed herein that are based on these biomarkers may serve in the point-of-care diagnostics that allow for rapid and inexpensive determination of a Th1 immune status, which may result in significant cost savings to the medical system as subjects with an undesirable Th1 immune response can be exposed to therapeutic agents that are suitable for enhancing or depleting the Th1 immune response in the subject as necessary.

Using the methods described herein, a number of biomarkers have been identified that are particularly useful for determining the Th1 immune status of a subject. These biomarkers are referred to herein as "Th1 immune status biomarkers". As used herein, the term "Th1 immune status biomarker" refers to a biomarker of the subject, generally a biomarker of the subject's immune system, which is altered, or whose level of expression is altered, as part of a Th1 immune response. The Th1 immune status biomarkers are suitably expression products of genes (also referred to interchangeably herein as "Th1 immune response biomarker genes"), including polynucleotide and polypeptide expression products. As used herein, polynucleotide expression products of Th1 immune status biomarker genes are referred to herein as "Th1 immune status biomarker polynucleotides." Polypeptide expression products of the Th1 immune response biomarker genes are referred to herein as "Th1 immune status biomarker polypeptides."

The at least one Th1 immune status biomarker of the present invention suitably comprises PD-L2. The native human PD-L2 amino acid sequence is set forth in SEQ ID NO: 1, and is encoded by the nucleic acid sequence set forth in SEQ ID NO: 3. Another Th1 immune status biomarker that can optionally be used in the methods of the invention is PD-L1. The native human PD-L1 amino acid sequence is set forth in SEQ ID NO: 2, and is encoded by the nucleic acid sequence set forth in SEQ ID NO: 4.

Of the above Th1 immune status biomarkers, the PD-L2 polypeptide has been found to have strong diagnostic performance on its own for detecting Th1 immune status (as measured, for example, using FACS analysis by measuring percentage of PD-L2⁺ IEC-interacting cells (e.g., APCs or tumor cells)). Thus, in specific embodiments the PD-L2 biomarker may be used either by itself or in combination with other Th1 immune status biomarkers for the determination of the indicator. Suitably, in these embodiments, a biomarker value is measured or derived for the PD-L2 biomarker and optionally another Th1 immune status biomarker(s) (e.g., PD-L1) to determine the indicator.

The present inventors have also determined that other Th1 immune status biomarkers have strong diagnostic performance when used in combination with the PD-L2 biomarker. In advantageous embodiments, pairs of Th1 immune status biomarkers have been identified that can be used to determine the indicator. Accordingly, in representative examples of this type, and as described in detail below, an indicator is determined that correlates to a ratio of Th1 immune status biomarkers, which can be used in determining the Th1 immune status of a subject.

Thus, specific protein products are disclosed herein as Th1 immune response biomarkers that provide a means for determining the Th1 immune status of a subject. Evaluation of these Th1 immune status biomarkers through analysis of their levels in a subject, or in a sample obtained from a subject, provides a measured or derived biomarker value for determining an indicator that can be used for assessing the Th1 immune status in a subject.

### 2.2 Sample preparation

Generally, a sample is processed prior to Th1 immune status biomarker detection or quantification. For example, proteins and/or nucleic acids may be extracted, isolated, and/or purified from a sample prior to analysis. Various protein, DNA, and/or mRNA extraction and purification techniques are well known to those skilled in the art. Processing may include centrifugation, ultracentrifugation, ethanol precipitation, filtration, fractionation, resuspension, dilution, concentration, *etc.* In some embodiments, the methods taught above and elsewhere herein provide analysis (*e*.*g*., quantification of protein biomarkers) from raw sample (*e*.*g*., biological fluid such as blood, serum, *etc*.) without or with limited processing.

Furthermore, a sample can be processed prior to Th1 immune status biomarker detection or quantification in order to purify or enrich the sample for a particular fraction or cell type of interest. For example, the sample can be enriched for IEC-interacting cells (*e*.*g*., APCs or tumor cells), or a particular subset of IEC-interacting cells (*e*.*g*., dendritic cells, macrophages, monocytes or a combination thereof). In preferred embodiments, the sample is enriched for dendritic cells (*e*.*g*., CD11c⁺ dendritic cells) prior to Th1 immune status biomarker detection or quantification. Methods for enriching biological samples for a particular cell type are well known in the art. For example, dendritic cells may be isolated by differential gradient separation using, for example, Ficoll-hypaque or sucrose gradient solutions for cell separations, followed by ammonium chloride or hypotonic lysis of remaining contaminating erythrocytes ("Cell Biology: A Laboratory Handbook", Volumes I-III Cellis, J. E., ed. (1994); "Current Protocols in Immunology" Volumes I-III Coligan J. E., ed. (1994); Stites et al. (eds)).

Sample preparation methods may comprise steps of homogenizing a sample in a suitable buffer, removal of contaminants and/or assay inhibitors, adding a Th1 immune status biomarker capture reagent (*e*.*g*., a magnetic bead which is linked to a moiety that can specifically bind to a target Th1 immune status biomarker), incubated under conditions that promote the association of the target biomarker with the capture reagent to produce a target biomarker: capture reagent complex, and incubating the target biomarker: capture complex under target biomarker-release conditions. In some embodiments, multiple Th1 immune status biomarkers are isolated in each round of isolation by adding multiple Th1 immune status biomarkers capture reagents (*e*.g*.*, specific to the desired biomarkers) to the solution. For example, in an illustrative embodiment in which the biomarker is a nucleic acid, multiple Th1 immune status biomarker capture reagents, each comprising an oligonucleotide specific for a different target Th1 immune status biomarker can be added to the sample for isolation of multiple Th1 immune status biomarkers. It is contemplated that the methods encompass multiple experimental designs that vary both in the number of capture steps and in the number of target Th1 immune status biomarkers captured in each capture step. In some embodiments, capture reagents are molecules, moieties, substances, or compositions that preferentially (*e*.*g*., specifically and selectively) interact with a particular biomarker sought to be isolated, purified, detected, and/or quantified. Any capture reagent having desired binding affinity and/or specificity to the particular Th1 immune status biomarker can be used in the present technology. For example, the capture reagent can be a macromolecule such as a peptide, a protein (*e*.*g*., an antibody or receptor), an oligonucleotide, a nucleic acid, (*e*.*g*., nucleic acids capable of hybridizing with the Th1 immune status biomarkers), vitamins, oligosaccharides, carbohydrates, lipids, or small molecules, or a complex thereof. As illustrative and non-limiting examples, an avidin target capture reagent may be used to isolate and purify targets comprising a biotin moiety, an antibody may be used to isolate and purify targets comprising the appropriate antigen or epitope, and an oligonucleotide may be used to isolate and purify a complementary oligonucleotide.

Any nucleic acids, including single-stranded and double-stranded nucleic acids, that are capable of binding, or specifically binding, to a target Th1 immune status biomarker can be used as the capture reagent. Examples of such nucleic acids include DNA, RNA, aptamers, peptide nucleic acids, and other modifications to the sugar, phosphate, or nucleoside base. Thus, there are many strategies for capturing a target and accordingly many types of capture reagents are known to those in the art.

In addition, Th1 immune status biomarker capture reagents may comprise a functionality to localize, concentrate, aggregate, *etc.* the capture reagent and thus provide a way to isolate and purify the target Th1 immune status biomarker when captured (*e*.*g*., bound, hybridized, *etc*.) to the capture reagent (*e.g.,* when a target:capture reagent complex is formed). For example, in some embodiments the portion of the capture reagent that interacts with the Th1 immune status biomarker (*e.g.,* a polypeptide) is linked to a solid support (*e.g.,* a bead, surface, resin, column, and the like) that allows manipulation by the user on a macroscopic scale. Often, the solid support allows the use of a mechanical means to isolate and purify the target:capture reagent complex from a heterogeneous solution. For example, when linked to a bead, separation is achieved by removing the bead from the heterogeneous solution, *e.*g., by physical movement. In embodiments in which the bead is magnetic or paramagnetic, a magnetic field is used to achieve physical separation of the capture reagent (and thus the target Th1 immune status biomarker) from the heterogeneous solution.

### 2.3 Evaluation of Th1 immune status biomarker polypeptides

In order to obtain the biomarker value, the Th1 immune status biomarkers may be quantified or detected using any suitable technique that is known in the art. In specific embodiments, the Th1 immune status biomarkers are quantified using reagents that determine the level, abundance or amount of individual Th1 immune status biomarkers. Non-limiting reagents of this type include reagents for use in protein-based and nucleic acid-based assays.

Th1 immune status biomarker expression may be evaluated at the level of protein expression, either by demonstration of the presence of the protein, or by one or more known functional properties of the biomarker. For example, anti-PD-L2 antibodies for use in PD-L2-specific protein detection are described in U.S. Pat. No. 7,709,214; U.S. patent application Ser. No. 2009/296,392; and European Pat. No. 1537878. The antibodies bind both native and denatured PD-L2 protein and may be detected by several well-known assays in the art, including enzyme linked immunosorbent assays (ELISA), radioimmunoassays (RIA), light emission immunoassays, Western blot analysis, immunofluorescence assays, immunohistochemistry and fluorescence activated cell sorting (FACS) analysis.

ELISA and RIA follow similar principles for detection of specific antigens. By way of an illustrative example, PD-L2 can be measured using RIA by way of a PD-L2-specific antibody that is radioactively labeled, typically with ¹²⁵I. In ELISA assays a PD-L2-specific antibody is chemically linked to an enzyme. PD-L2-specific capturing antibody is immobilized onto a solid support. Unlabeled specimens, e.g., protein extracts from biological samples are then incubated with the immobilized antibody under conditions where non-specific binding is blocked, and unbound antibody and/or protein removed by washing. Bound PD-L2 is detected by a second PD-L2 specific labeled antibody. Antibody binding is measured directly in RIA by measuring radioactivity, while in ELISA binding is detected by a reaction converting a colourless substrate into a coloured reaction product, as a function of linked-enzyme activity. Changes can thus readily be detected by spectrophotometry (Janeway C. A. *et al.* (1997). "Immunobiology" 3.sup.rd Edition, Current Biology Ltd., Garland Publishing Inc.; "Cell Biology: A Laboratory Handbook", Volumes I-III Cellis, J. E., ed. (1994); "Current Protocols in Immunology" Volumes I-III Coligan J. E., ed. (1994); Stites *et al.* (eds)). Both assays therefore provide a means of quantification of PD-L2 protein content in a biological sample.

Protein biomarker expression may also be detected *via* light emission immunoassays. Much like ELISA and RIA, in light emission immunoassays the biological sample/protein extract to be tested is immobilized on a solid support, and probed with a specific label, labeled anti-PD-L2 antibody. The label, in turn, is luminescent, and emits light upon binding, as an indication of specific recognition. Luminescent labels include substances that emit light upon activation by electromagnetic radiation, electro chemical excitation, or chemical activation and may include fluorescent and phosphorescent substances, scintillators, and chemiluminescent substances. The label can be a part of a catalytic reaction system such as enzymes, enzyme fragments, enzyme substrates, enzyme inhibitors, coenzymes, or catalysts; part of a chromogen system such as fluorophores, dyes, chemiluminescers, luminescers, or sensitizers; a dispersible particle that can be non-magnetic or magnetic, a solid support, a liposome, a ligand, a receptor, a hapten radioactive isotope, and so forth (U.S. Pat. Nos. 6,410,696, U.S. Pat. No. 4,652,533 and European Patent Application No. 0,345,776), and provide an additional, highly sensitive method for detection of PD-L2 protein expression.

Western blot analysis is another means of assessing Th1 immune status biomarker polypeptide content in a biological sample. Protein extracts from biological samples of IEC-interacting cells (e.g., APCs, such as dendritic cells, or tumor cells), are solubilized in a denaturing ionizing environment, and aliquots are applied to polyacrylamide gel matrixes. Proteins separate based on molecular size properties as they migrate toward the anode. Antigens are then transferred to nitrocellulose, PVDF or nylon membranes, followed by membrane blocking to minimize non-specific binding. Membranes are probed with antibodies directly coupled to a detectable moiety, or are subsequently probed with a secondary antibody containing the detectable moiety. Typically, the enzymes horseradish peroxidase or alkaline phosphatase are coupled to the antibodies, and chromogenic or luminescent substrates are used to visualize activity (Harlow E. et al., (1998) Immunoblotting. In Antibodies: A Laboratory Manual, pp. 471-510 CSH Laboratory, cold Spring Harbor, N.Y. and Bronstein I. et al. (1992) Biotechniques 12: 748-753).

Unlike RIA, ELISA, light emission immunoassays and immunoblotting, which quantify protein biomarker content in whole samples, immunofluorescence/immunocytochemistry may be used to detect proteins in a cell-specific manner, though quantification is compromised.

As described above, IEC-interacting cells (*e*.*g*., APCs, including dendritic cells, or tumor cells) may be isolated or enriched by methods known in the art. Isolation or enrichment of IEC-interacting cells (*e*.*g*., APCs or tumor cells) refers to a process wherein the percentage of IEC-interacting cells (*e*.*g*., APCs or tumor cells) is increased (relative to the percentage in the sample before the enrichment procedure). Purification is one example of enrichment. In certain embodiments, the increase in the number of IEC-interacting cells (*e*.*g*., APCs or tumor cells) of the invention as a percentage of cells in the enriched sample, relative to the sample prior to the enrichment procedure, is at least 25-, 50-, 75-, 100-, 150-, 200-, 250-, 300-, 350-fold, and suitably is 100-200-fold. In specific embodiments, antibodies to surface markers on IEC-interacting cells (*e*.*g*., APCs or tumor cells) may be attached to a solid support to allow for separation. Procedures for separation may include magnetic separation, using antibody magnetic beads (*e*.*g*., Miltenyi^{™} beads), affinity chromatography, "panning" with antibody attached to a solid matrix or any other convenient technique such as Laser Capture Microdissection. In specific embodiments, the APCs are enriched using an antibody that is specific for CD11c, which antibody is conjugated to a magnetic bead, and a magnetic cell separation device to separate out the CD11c⁺ cells. Other techniques providing particularly accurate separation include FACS. Once cells are deposited on slides, they may be fixed, and probed with labeled antibody for detection of Th1 immune status biomarker in a cell specific fashion.

Antibodies specific for a Th1 immune status biomarker, for example, anti-PD-L2 antibodies, may be directly conjugated to fluorescent markers, including fluorescein, FITC, rhodamine, Texas Red, Cy3, Cy5, Cy7, and other fluorescent markers, and viewed in a fluorescent microscope, equipped with the appropriate filters. Antibodies may also be conjugated to enzymes, which upon addition of an appropriate substrate commence a reaction providing a coloured precipitate over cells with detected PD-L2 protein. Slides may then be viewed by standard light microscopy. Alternatively, primary antibodies specific for PD-L2 may be further bound to secondary antibodies conjugated to the detectable moieties. Cell surface expression can be thus assessed, and the addition of cell permeabilization solutions, such as Triton-X and saponin may be applied to facilitate reagent penetration within cell cytoplasms ("Cell Biology: A Laboratory Handbook", Volumes 1-111 Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, Conn. (1994); Mishell and Shiigi (eds), "Selected Methods in Cellular Immunology", W. H. Freeman and Co., New York (1980)).

Immunohistochemistry is quite similar to immunofluorescence or immunocytochemistry, in principle, however tissue specimens are probed with PD-L2 antibody, for example, as opposed to cell suspensions. Biopsy specimens are fixed and processed and optionally embedded in paraffin, sectioned if needed, providing cell or tissue slides subsequently probed with heparanase specific antibodies. Alternatively, frozen tissue may be sectioned on a cryostat, with subsequent antibody probing, obviating fixation-induced antigen masking. Antibodies, as in immunofluorescence or immunocytochemistry, are coupled to a detectable moiety, either fluorescent, or enzyme-linked, and are used to probe tissue sections by methods described for immunofluorescence, and are subsequently visualized by fluorescent or confocal microscopy, depending upon the detection method employed. Visualization of a reaction product precipitate may be viewed by standard light microscopy, if an enzymatic detectable moiety was utilized, following development of the reaction product ("Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, Conn. (1994); Mishell and Shiigi (eds), "Selected Methods in Cellular Immunology", W. H. Freeman and Co., New York (1980)).

In specific embodiments, FACS analysis is used to assess Th1 immune status biomarker expression (*e*.*g*., PD-L2, and optionally PD-L1, expression). A general description of FACS apparatus and methods in provided in U.S. Pat. Nos. 4,172,227; 4,347,935; 4,661,913; 4,667,830; 5,093,234; 5,094,940; and 5,144,224. Cells are introduced into the FACS machine and are delivered *via* tubing into the FACS cell, which they pass through as single cells. A laser beam is directed at the FACS cell, and forward laser scatter is collected by a photodiode, side laser scatter is directed to a PMT tube via a lens, directed to PMT1. Specific filters direct fluorescence from the side scatter to other PMT tubes for multivariate analysis. Side laser scatter is a reflection of cell size and granularity, and may be used to identify cell populations in mixed samples. Cells labeled with fluorescent anti-PD-L2 antibody may be detected by laser excitation and collection *via* PMT tubes, which can be identified for cell type via size and granularity, or *via* incorporation of additional cell surface markers for identification, as for example disclosed above. Typically, FACS analysis is used for determination of cell surface expression of a particular protein (e.g., PD-L2, and optionally PD-L1, expression), and hence specific antibodies may be utilized for probing detection of cell surface biomarker expression in APC populations (e.g., PD-L2, and optionally PD-L1, expression on the surface of dendritic cells). Specific APC subtypes (e.g., CD11c⁺ dendritic cells) expressing surface PD-L2 protein and optionally PD-L1 may be ascertained by size and granularity characteristics, or alternatively by co-staining with additional cell surface marker proteins.

In specific embodiments, protein-capture arrays that permit simultaneous detection and/or quantification of a large number of proteins are employed. For example, low-density protein arrays on filter membranes, such as the universal protein array system (Ge, 2000 Nucleic Acids Res. 28(2):e3) allow imaging of arrayed antigens using standard ELISA techniques and a scanning charge-coupled device (CCD) detector. Immuno-sensor arrays have also been developed that enable the simultaneous detection of clinical analytes. It is now possible using protein arrays, to profile protein expression in bodily fluids, such as in sera of healthy or diseased subjects, as well as in subjects pre- and post-drug treatment.

Exemplary protein capture arrays include arrays comprising spatially addressed antigen-binding molecules, commonly referred to as antibody arrays, which can facilitate extensive parallel analysis of numerous proteins defining a proteome or subproteome. Antibody arrays have been shown to have the required properties of specificity and acceptable background, and some are available commercially (*e*.*g*., BD Biosciences, Clontech, Bio-Rad and Sigma). Various methods for the preparation of antibody arrays have been reported (see, *e.g.,* Lopez et al., 2003 J. Chromatogram. B 787:19-27; Cahill, 2000 Trends in Biotechnology 7:47-51; U.S. Pat. App. Pub. 2002/0055186; U.S. Pat. App. Pub. 2003/0003599; PCT publication WO 03/062444; PCT publication WO 03/077851; PCT publication WO 02/59601; PCT publication WO 02/39120; PCT publication WO 01/79849; PCT publication WO 99/39210). The antigen-binding molecules of such arrays may recognize at least a subset of proteins expressed by a cell or population of cells, illustrative examples of which include growth factor receptors, hormone receptors, neurotransmitter receptors, catecholamine receptors, amino acid derivative receptors, cytokine receptors, extracellular matrix receptors, antibodies, lectins, cytokines, serpins, proteases, kinases, phosphatases, ras-like GTPases, hydrolases, steroid hormone receptors, transcription factors, heat-shock transcription factors, DNA-binding proteins, zinc-finger proteins, leucine-zipper proteins, homeodomain proteins, intracellular signal transduction modulators and effectors, apoptosis-related factors, DNA synthesis factors, DNA repair factors, DNA recombination factors and cell-surface antigens.

Individual spatially distinct protein-capture agents are typically attached to a support surface, which is generally planar or contoured. Common physical supports include glass slides, silicon, microwells, nitrocellulose or PVDF membranes, and magnetic and other microbeads.

Particles in suspension can also be used as the basis of arrays, providing they are coded for identification; systems include color coding for microbeads (*e*.*g*., available from Luminex, Bio-Rad and Nanomics Biosystems) and semiconductor nanocrystals (*e*.*g*., QDots^{™}, available from Quantum Dots), and barcoding for beads (UltraPlex^{™}, available from Smartbeads) and multimetal microrods (Nanobarcodes^{™} particles, available from Surromed). Beads can also be assembled into planar arrays on semiconductor chips (*e*.*g*., available from LEAPS technology and BioArray Solutions). Where particles are used, individual protein-capture agents are typically attached to an individual particle to provide the spatial definition or separation of the array. The particles may then be assayed separately, but in parallel, in a compartmentalized way, for example in the wells of a microtiter plate or in separate test tubes.

In operation, a protein sample, which is optionally fragmented to form peptide fragments (see, *e*.*g*., U.S. Pat. App. Pub. 2002/0055186), is delivered to a protein-capture array under conditions suitable for protein or peptide binding, and the array is washed to remove unbound or non-specifically bound components of the sample from the array. Next, the presence or amount of protein or peptide bound to each feature of the array is detected using a suitable detection system. The amount of protein bound to a feature of the array may be determined relative to the amount of a second protein bound to a second feature of the array. In certain embodiments, the amount of the second protein in the sample is already known or known to be invariant.

In specific embodiments, the Th1 immune status biomarker is a target polypeptide whose level is measured using at least one antigen-binding molecule that is immuno-interactive with the target polypeptide. In these embodiments, the measured level of the target polypeptide is normalized to the level of a reference polypeptide. Suitably, the antigen-binding molecule is immobilized on a solid or semi-solid support. In illustrative examples of this type, the antigen-binding molecule forms part of a spatial array of antigen-binding molecule. In some embodiments, the level of antigen-binding molecule that is bound to the target polypeptide is measured by immunoassay (*e*.*g*., using an ELISA).

Demonstration of the absence or presence of biomarker activity within a sample is an additional means of distinguishing IEC-interacting cells (*e*.*g*., APCs or tumor cells) expressing a specific Th1 immune status biomarker versus non-expressing cell populations.

### 2.4 Evaluation of PD-L2 biomarker clustering

The present inventors have also discovered that clustering of PD-L2 on the cell surface of IEC-interacting cells (*e*.*g*., APCs or tumor cells) is indicative of a normal or elevated Th1 immune response. Thus, in some embodiments the biomarker value of the Th1 immune status biomarker is indicative of the level or abundance of PD-L2, as determined by analyzing the PD-L2 clustering on the cell surface of an APC (*e*.*g*., a dendritic cell).

There are a number of widely available assays to detect clustering of PD-L2 on the cell surface of an APC (*e*.*g*., a dendritic cell). For example, PD-L2 ligands and/or PD-L2-specific antibodies can be labeled, and these labels detected to visualize clustering of PD-L2. In one example of this type of assay, a dendritic cell comprising the PD-L2 is contacted with a PD-L2-specific antibody, and a fluorescently labeled secondary antibody that binds to the PD-L2-specific antibody. Using confocal scanning laser microscopy, fluorescence emitted from the secondary antibody can be detected to identify the location of the PD-L2. (Van Steensel, et al., 1995. J. Cell Sci. 108: 3003-3011). In another example, a cell comprising PD-L2 on the cell surface is contacted with a labeled ligand of the cell surface PD-L2 and cell surface PD-L2 clustering analyzed by super resolution microscopy, as described for example by Kaufmann et al. (2011. J. Microsc. 242(1):46-54), Huber et al. (2011. PLoS One. 7(9):e44776), Wang et al. (2014. Biochim. Biophys. Acta. 1838(4):1191-1198), and Sams et al. (2014. J Biomed. Opt. 19(1):011021).

Alternatively, cell surface PD-L2 clustering is analyzed by *in situ* proximity assay as described for example by Bellucci et al. (2014. Methods Mol. Biol. 1174:397-405), Barros et al. (2014. Breast Cancer Res. Treat. 144(2):273-85) and Pacchiana et al. (2014. Histochem. Cell. Biol. 142(5):593-60).

In other embodiments, FRET and FRAP microscopy can be employed to analyze PD-L2 clustering, as described for example by Wallrabe et al. (2003. Biophys. J. 85(1):559-571), Wallrabe et al. (2003. J. Biomed. Opt. 8(3):339-346) and de Heus et al. (2013. Methods Cell. Biol. 117:305-321).

Other methods of analyzing PD-L2 clustering include: image correlation spectroscopy as described for example by Petersen et al. (1998. Faraday Discuss. (111):289-305), Kozer et al. (2013. Mol. Biosyst. 9(7):1849-1863), and Ciccotosto et al. (2013. Biophys. J. 104(5):1056-1064); electric field analysis, as described for example by Giugni et al. (1987. J. Cell. Biol. 104(5):1291-1297), and Zhang et al. (2011. PLoS One. 6(10):e26805), electron microscopy, as described for example by Plowman et al. (2005. Proc. Natl. Acad. Sci. USA. 102(43):15500-15505), and D'Amico et al. (2008. Micron. 39(1):1-6); electron cryotomography, as described for example by Gold et al. (2014. Nat. Commun. 5:4129); nanoparticle (NP) immunolabelling in combination with plasmon coupling microscopy (PCM), as described for example by Wang et al. (2012. Nano. Lett. 12(6):3231-3237) and Rong et al. (2012. PLoS One. 7(3):e34175); enzyme-mediated activation of radical source (EMARS) analysis, as described for example by Miyagawa-Yamaguchi et al. (2014. PLoS One. 9(3):e93054) and Kotani et al. (2008. Proc. Natl. Acad. Sci. USA. 105(21):7405-7409); and quantum dots analysis, as described for example by Li et al. (2010. Biophys. J. 98(11):2554-2563).

In other embodiments, a flow cytometer equipped with a doublet discriminator is used to determine the distribution of a labeled PD-L2 ligand on a single cell. The distribution of the label on the cell is then correlated with the formation of PD-L2 clusters to thereby determine the receptor element clustering status of the cell. An exemplary method of this type is disclosed in U.S. Pat. Appl. Pub. No. 2016/0003840.

Numerous ligands with specificity for PD-L2 are known, which can be used for clustering analysis. Many of these are also useful as therapeutic agents in accordance with the present invention. For example, any suitable antibody that binds a PD-L2 polypeptide is contemplated for use in the practice of the present invention. Non-limiting examples of such antibodies are listed above.

In specific embodiments, the antibody comprises an Fc region of an immunoglobulin. Alternatively, or in addition, the antibody is a multivalent (*e*.*g*., bivalent) antibody.

Any PD-L2 ligand is suitable for use in these embodiments of the invention, including the following classes of ligand: protein, small organic molecule, carbohydrates (including polysaccharides), polynucleotide, lipids, *etc.* Representative examples of such ligands include PD-1 polypeptides, galectin-9 polypeptides, and repulsive guidance molecule b (RGMb).

### 2.5 Evaluation of Th1 immune status biomarker nucleic acids

In some embodiments, biomarker expression is monitored by determining biomarker nucleic acid transcript levels. RNA may be extracted from biological samples *via* a number of standard techniques (see Current Protocols in Molecular Biology" Volumes I-III Ausubel, R. M., ed. (1994); Ausubel et al., "Current Protocols in Molecular Biology", John Wiley and Sons, Baltimore, Md. (1989)). Guanidium-based methods for cell lysis enabling RNA isolation, with subsequent cesium chloride step gradients for separation of the RNA from other cellular macromolecules, followed by RNA precipitation and resuspension, is an older, less commonly employed method of RNA isolation (Glisin, Ve. et al., (1973) Biochemistry 13: 2633). Alternatively, RNA may be isolated in a single step procedure (U.S. Pat. No. 4,843,155, and Puissant, C. And Houdebine L. M. (1990) Biotechniques 8: 148-149). Single step procedures include the use of Guanidium isothiocyanate for RNA extraction, and subsequent phenol/chloroform/isoamyl alcohol extractions facilitating the separation of total RNA from other cellular proteins and DNA. Commercially available single-step formulations based on the above-cited principles may be employed, including, for example, the use of the TRIZOL reagent (Life Technologies, Gaithersburg, Md.).

Th1 immune status biomarker RNA/gene expression can be monitored *via* a number of other standard techniques, illustrative examples of which include Northern blot and dot blot analysis, primer extension, RNase protection, RT-PCR, *in-situ* hybridization and chip hybridization.

Specific Th1 immune status biomarker RNA sequences can be readily detected by hybridization of labeled probes to blotted RNA preparations extracted as above. In Northern blot analysis, fractionated RNA is subjected to denaturing agarose gel electrophoresis, which prevents RNA from assuming secondary structures that might inhibit size based separation. RNA is then transferred by capillary transfer to a nylon or nitrocellulose membrane support and may be probed with a labeled oligonucleotide probe complementary to the biomarker sequence (Alwine, et al. (1977). Proc. Natl. Acad. Sci. USA 74: 5350-5354 and Current Protocols in Molecular Biology" Volumes I-III Ausubel, R. M., ed. (1994); Ausubel et al., "Current Protocols in Molecular Biology", John Wiley and Sons, Baltimore, Md. (1989)).

Alternatively, unfractionated RNA may be immobilized on a nylon or nitrocellulose membrane, and similarly probed for biomarker-specific expression, by Slot/Dot blot analysis. RNA slot/dot blots can be prepared by hand, or alternatively constructed using a manifold apparatus, which facilitates comparing hybridization signals by densitometry scanning (Chomczynski P. (1992) Anal. Biochem. 201: 134-139).

Primer extension is another means whereby quantification of the RNA may be accomplished. Primer extension provides an additional benefit in mapping the 5' terminus of a particular RNA, by extending a primer using the enzyme reverse transcriptase. In this case, the primer is an oligonucleotide (or restriction fragment) complementary to a portion of the biomarker mRNA. The primer is end-labeled, and is allowed to hybridize to template biomarker mRNA. Once hybridized, the primer is extended by addition of reverse transcriptase, and incorporation of unlabeled deoxynucleotides to for a single-stranded DNA complementary to template biomarker mRNA. DNA is then analyzed on a sequencing gel, with the length of extended primer serving to map the 5' position of the mRNA, and the yield of extended product reflecting the abundance of RNA in the sample (Jones et al., (1985) Cell. 42: 559-572 and Mierendorf R. C. And Pfeffer, D. (1987). Methods Enzymol. 152: 563-566).

RNase protection assays provide a highly sensitive means of quantifying biomarker RNA, even in low abundance. In protection assays, sequence-specific hybridization of ribonucleotide probes complementary to biomarker RNA, with high specific activity are generated, and hybridized to sample RNA. Hybridization reactions are then treated with ribonuclease to remove free probe, leaving intact fragments of annealed probe hybridized to homologous biomarker sequences in sample RNA. Fragments are then analyzed by electrophoresis on a sequencing gel, when appropriately-sized probe fragments are visualized (Zinn K. et al., (1983) Cell. 34: 865-879 and Melton S. A., et al., (1984). Nucl. Acids Res. 12: 7035-7056).

RT-PCR is another means by which biomarker expression may be analyzed. RT-PCR employs the use of reverse transcriptase to prepare cDNA from RNA samples, using deoxynucleotide primers complementary to the biomarker mRNA. Once the cDNA is generated, it is amplified through the polymerase chain reaction, by the addition of deoxynucleotides and a DNA polymerase that functions at high temperatures. Through repetitive cycles of primer annealing, incorporation of deoxynucleotides facilitating cDNA extension, followed by strand denaturation, amplification of the desired sequence occurs, yielding an appropriately sized fragment that may be detected by agarose gel electrophoresis. Optimal reverse transcription, hybridization, and amplification conditions will vary depending upon the sequence composition and length(s) of the primers and target(s) employed, and the experimental method selected by the practitioner. Various guidelines may be used to select appropriate primer sequences and hybridization conditions (see, *e.g.,* Sambrook et al., 1989, Molecular Cloning, A Laboratory Manual, (Volumes 1-3) Cold Spring Harbor Press, N.Y.; and Ausubel et al., 1989, Current Protocols in Molecular Biology, Green Publishing Associates and Wiley Interscience, N.Y.).

*In-situ* hybridization provides may be used for detecting and localizing cell/tissue specific biomarker RNA expression. Labeled anti-sense RNA probes are hybridized to mRNAs in cells singly, or in processed tissue slices, which are immobilized on microscope glass slides (In Situ Hybridization: Medical Applications (eds. G. R. Coulton and J. de Belleroche), Kluwer Academic Publishers, Boston (1992); In Situ Hybridization: In Neurobiology; Advances in Methodology (eds. J. H. Eberwine, K. L. Valentino, and J. D. Barchas), Oxford University Press Inc., England (1994); and In Situ Hybridization: A Practical Approach (ed. D. G. Wilkinson), Oxford University Press Inc., England (1992)). Numerous non-isotopic systems have been developed to visualize labeled DNA probes including; a) fluorescence-based direct detection methods, b) the use of digoxigenin- and biotin-labeled DNA probes coupled with fluorescence detection methods, and c) the use of digoxigenin-and biotin-labeled DNA probes coupled with antibody-enzyme detection methods. When fluorescence-labeled anti-sense RNA probes are hybridized to cellular RNA, the hybridized probes can be viewed directly using a fluorescence microscope. Direct fluorochrome-labelling of the nucleic acid probes eliminate the need for multi-layer detection procedures (e.g., antibody-based-systems), which allows fast processing and also reduces non-specific background signals, hence providing a versatile and highly sensitive means of identifying biomarker gene expression.

Chip hybridization utilizes biomarker specific oligonucleotides attached to a solid substrate, which may consist of a particulate solid phase such as nylon filters, glass slides or silicon chips (Schena et al. (1995) Science. 270:467-470) designed as a microarray. Microarrays are known in the art and consist of a surface to which probes that correspond in sequence to gene products (such as cDNAs) can be specifically hybridized or bound at a known position for the detection of biomarker gene expression.

Quantification of the hybridization complexes is well known in the art and may be achieved by any one of several approaches. These approaches are generally based on the detection of a label or marker, such as any radioactive, fluorescent, biological or enzymatic tags or labels of standard use in the art. A label can be applied to either the oligonucleotide probes or the RNA derived from the biological sample.

In general, mRNA quantification is suitably effected alongside a calibration curve so as to enable accurate mRNA determination. Furthermore, quantifying transcript(s) originating from a biological sample is preferably effected by comparison to a normal sample, which sample is characterized by normal expression pattern of the examined transcript(s).

### 2.6 Deriving biomarker values

Biomarker values can be measured biomarker values, which are values of biomarkers directly measured for the subject, or alternatively could be "derived" biomarker values, which are values that have been derived from one or more measured biomarker values, for example by applying a function to the one or more measured biomarker values. As used herein, biomarkers to which a function has been applied are referred to as "derived biomarkers."

The biomarker values may be determined in any one of a number of ways that are well known in the art. For example, a comprehensive description of biomarker value determination can be found in Intl. Pat. Pub. No. WO 2015/117204. In one example, the process of determining biomarker values can include measuring the biomarker values, for example by performing tests on the subject or on sample(s) obtained from the subject.

More typically, however, the step of determining the biomarker values includes having an electronic processing device receive or otherwise obtain biomarker values that have been previously measured or derived. This could include for example, retrieving the biomarker values from a data store such as a remote database, obtaining biomarker values that have been manually input, using an input device, or the like. Suitably, the indicator may be determined using a combination of a plurality of biomarker values, the indicator being at least partially indicative of Th1 immune status. Assuming the method is performed using an electronic processing device, an indication of the indicator is optionally displayed or otherwise provided to the user.

In some embodiments, biomarker values are combined, for example by adding, multiplying, subtracting, or dividing biomarker values to determine an indicator value. This step is performed so that multiple biomarker values can be combined into a single indicator value, providing a more useful and straightforward mechanism for allowing the indicator to be interpreted and hence used in determining the Th1 immune status of the subject.

It will be understood that in this context, the biomarkers used within the above-described method can define a biomarker profile for Th1 immune status, which includes a minimal number of biomarkers (*e*.*g*., at least one biomarker), whilst maintaining sufficient performance to allow the biomarker profile to be used in making a clinically relevant determination. Minimizing the number of biomarkers used minimizes the costs associated with performing diagnostic or prognostic tests and in the case of polypeptide biomarkers, allows the test to be performed utilizing relatively straightforward techniques such as fluorescence-activated cell sorting (FACS) and immunohistochemistry, and allowing the test to be performed rapidly in a clinical environment. In this regard, the indication provided by the methods described herein could be a graphical or alphanumeric representation of an indicator value. Alternatively, however, the indication could be the result of a comparison of the indicator value to predefined thresholds or ranges, or alternatively could be an indication of the Th1 immune status.

Furthermore, producing a single indicator value allows the results of the test to be easily interpreted by a clinician or other medical practitioner, so that test can be used for reliable diagnosis in a clinical environment.

Solely by way of an illustration, the indicator-determining methods suitably include determining at least one biomarker value, wherein the biomarker value is a value measured or derived for at least one Th1 immune status biomarker of the subject and is at least partially indicative of a concentration or abundance of the Th1 immune status biomarker in a sample taken from the subject, and wherein the at least one Th1 immune status biomarker comprises PD-L2 of IEC-interacting cells (*e*.*g*., APCs or tumor cells). Suitably, the Th1 immune status biomarker profile further comprises PD-L1 of IEC-interacting cells (*e*.*g*., APCs or tumor cells) as a Th1 immune status biomarker. The biomarker values are typically used to determine an indicator for use in determining the Th1 immune status of a subject. In some embodiments, the indicator is indicative of a ratio of concentrations of a pair of Th1 immune status biomarkers (*e*.*g*., PD-L2 and PD-L1). Thus, if the biomarker values denote the concentrations of the Th1 immune status biomarker, then the derived biomarker value will typically (although not exclusively) be based on a ratio of the biomarker values.

The derived biomarker value is then used to determine the indicator, either by using the derived biomarker value as an indicator value, or by performing additional processing, such as comparing the derived biomarker value to a reference or the like, as generally known in the art and as described in more detail below.

The derived biomarker values could be combined using a combining function such as an additive model; a linear model; a support vector machine; a neural network model; a random forest model; a regression model; a genetic algorithm; an annealing algorithm; a weighted sum; a nearest neighbor model; and a probabilistic model. In some embodiments, biomarker values are measured or derived for PD-L2 and for PD-L1, and the indicator is determined by combining the biomarker values. In some embodiments, the indicator is compared to an indicator reference, with a Th1 immune status being determined in accordance with results of the comparison. The indicator reference may be derived from indicators determined for a number of individuals in a reference population. The reference population typically includes individuals having different characteristics, such as a plurality of individuals of different sexes; and/or ethnicities, with different groups being defined based on different characteristics, with the subject's indicator being compared to indicator references derived from individuals with similar characteristics. The reference population can also include a plurality of healthy individuals, a plurality of individuals known to have an enhanced Th1 immune status, a plurality of individuals known to have a reduced or deficient Th1 immune status, a plurality of individuals showing clinical signs of a metastatic cancer, a plurality of individuals showing clinical signs of a pathogenic infection (e.g., malaria),a plurality of individuals showing clinical signs of an autoimmune disease (e.g., irritable bowel syndrome).

In specific embodiments, the indicator-determining methods of the present invention are performed using at least one electronic processing device, such as a suitably programmed computer system or the like. In this case, the electronic processing device typically obtains at least one measured biomarker value, either by receiving this from a measuring or other quantifying device, or by retrieving these from a database or the like. The processing device then determines the indicator by any suitable means, for example, by calculating a value that is indicative of a ratio of concentrations of a first Th1 immune status biomarker and a second Th1 immune status biomarker. In one aspect, the present invention encompasses an apparatus comprising such electronic processing device(s).

The processing device can then generate a representation of the indicator, for example by generating a sign or alphanumeric indication of the indicator, a graphical indication of a comparison of the indicator to one or more indicator references or an alphanumeric indication of the Th1 immune status of the subject.

The indicator-determining methods of the present invention typically include obtaining a sample from a subject, who typically has at least one clinical sign of a Th1-related disease (for example, irritable bowel syndrome), wherein the sample includes one or more Th1 immune status biomarkers (*e*.*g*., PD-L2 and optionally PD-L1) and quantifying or otherwise assessing at least one (*e*.*g*., 1, 2, 3, 4, 5, 6, 7, 8, 9, 10 or more) of the Th1 immune status biomarkers within the sample to determine biomarker values. This can be achieved using any suitable technique, and will depend on the nature of the Th1 immune status biomarkers. Suitably, an individual measured or derived Th1 immune status biomarker value corresponds to the level, abundance or amount of a respective Th1 immune status biomarker or to a function that is applied to that level or amount. For example, if the indicator in some embodiments of the indicator-determining method of the present invention, which uses a plurality of Th1 immune status biomarkers, is based on a ratio of concentrations of a polypeptide, this process would typically include quantifying the polypeptide by any means known in the art, including immunofluorescence, or by a functional assay.

In some embodiments, the Th1 immune status of a subject is established by determining one or more Th1 immune status biomarker values, wherein an individual Th1 immune status biomarker value is indicative of a value measured or derived for a Th1 immune status biomarker in a subject or in a sample obtained from the subject. These biomarkers are referred to herein as "sample Th1 immune status biomarkers." In accordance with the present invention, a sample Th1 immune status biomarker corresponds to a reference Th1 immune status biomarker (also referred to herein as a "corresponding Th1 immune status biomarker"). By "corresponding Th1 immune status biomarker" is meant a Th1 immune status biomarker that is structurally and/or functionally similar to a reference Th1 immune status biomarker as set forth for example in SEQ ID NO:1 (PD-L2) and SEQ ID NO: 2 (PD-L1). Representative corresponding Th1 immune status biomarkers include expression products of allelic variants (same locus), homologues (different locus), and orthologues (different organism) of reference Th1 immune response biomarker genes. Nucleic acid variants of reference Th1 immune status biomarker genes and encoded Th1 immune status biomarker polypeptides can contain nucleotide substitutions, deletions, inversions and/or insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of a reference Th1 immune status polypeptide.

Corresponding Th1 immune status biomarkers include amino acid sequences that display substantial sequence similarity or identity to the amino acid sequence of a reference Th1 immune status biomarker polypeptide. In general, an amino acid sequence that corresponds to a reference amino acid sequence will display at least about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 97, 88, 89, 90, 91, 92, 93, 94, 95, 96, 97, 98, 99% or even up to 100% sequence similarity or identity to a reference amino acid sequence selected from SEQ ID NO: 1 or SEQ ID NO: 2, as summarized in Table 4.

In some embodiments, calculations of sequence similarity or sequence identity between sequences are performed as follows:
To determine the percentage identity of two amino acid sequences, or of two nucleic acid sequences, the sequences are aligned for optimal comparison purposes (*e*.*g*., gaps can be introduced in one or both of a first and a second amino acid or nucleic acid sequence for optimal alignment and non-homologous sequences can be disregarded for comparison purposes). In some embodiments, the length of a reference sequence aligned for comparison purposes is at least 30%, usually at least 40%, more usually at least 50%, 60%, and even more usually at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide at the corresponding position in the second sequence, then the molecules are identical at that position. For amino acid sequence comparison, when a position in the first sequence is occupied by the same or similar amino acid residue (*i*.*e*., conservative substitution) at the corresponding position in the second sequence, then the molecules are similar at that position.

The percentage identity between the two sequences is a function of the number of identical amino acid residues shared by the sequences at individual positions, taking into account the number of gaps, and the length of each gap, which need to be introduced for optimal alignment of the two sequences. By contrast, the percentage similarity between the two sequences is a function of the number of identical and similar amino acid residues shared by the sequences at individual positions, taking into account the number of gaps, and the length of each gap, which need to be introduced for optimal alignment of the two sequences.

The comparison of sequences and determination of percentage identity or percentage similarity between sequences can be accomplished using a mathematical algorithm. In certain embodiments, the percentage identity or similarity between amino acid sequences is determined using the Needleman and Wünsch, (1970, J. Mol. Biol. 48: 444-453) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In specific embodiments, the percent identity between nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. An non-limiting set of parameters (and the one that should be used unless otherwise specified) includes a Blossum 62 scoring matrix with a gap penalty of 12, a gap extend penalty of 4, and a frameshift gap penalty of 5.

In some embodiments, the percentage identity or similarity between amino acid or nucleotide sequences can be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al., (1990, J. Mol. Biol., 215: 403-10). BLAST nucleotide searches can be performed with the NBLAST program, score = 100, wordlength = 12 to obtain nucleotide sequences homologous to 53010 nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997, Nucleic Acids Res. 25: 3389-3402). When utilizing BLAST and Gapped BLAST programs, the default parameters of the respective programs (*e*.*g*., XBLAST and NBLAST) can be used.

Corresponding Th1 immune status biomarker polynucleotides also include nucleic acid sequences that hybridize to reference Th1 immune status biomarker polynucleotides, or to their complements, under stringency conditions described below. As used herein, the term "hybridizes under low stringency, medium stringency, high stringency, or very high stringency conditions" describes conditions for hybridization and washing. "Hybridization" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA, U pairs with A and C pairs with G. In this regard, the terms "match" and "mismatch" as used herein refer to the hybridization potential of paired nucleotides in complementary nucleic acid strands. Matched nucleotides hybridize efficiently, such as the classical A-T and G-C base pair mentioned above. Mismatches are other combinations of nucleotides that do not hybridize efficiently.

Guidance for performing hybridization reactions can be found in Ausubel *et al.,* (1998, *supra),* Sections 6.3.1-6.3.6. Aqueous and non-aqueous methods are described in that reference and either can be used. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M NaHPO₄ (pH 7.2), 7% SDS for hybridization at 65° C, and (i) 2 × SSC, 0.1% SDS; or (ii) 0.5% BSA, 1 mM EDTA, 40 mM NaHPO₄ (pH 7.2), 5% SDS for washing at room temperature. One embodiment of low stringency conditions includes hybridization in 6 × sodium chloride/sodium citrate (SSC) at about 45° C, followed by two washes in 0.2 × SSC, 0.1% SDS at least at 50° C (the temperature of the washes can be increased to 55° C for low stringency conditions). Medium stringency conditions include and encompass from at least about 16% v/v to at least about 30% v/v formamide and from at least about 0.5 M to at least about 0.9 M salt for hybridization at 42° C, and at least about 0.1 M to at least about 0.2 M salt for washing at 55° C. Medium stringency conditions also may include 1% Bovine Serum Albumin (BSA), 1 mM EDTA, 0.5 M NaHPO₄ (pH 7.2), 7% SDS for hybridization at 65° C, and (i) 2 × SSC, 0.1% SDS; or (ii) 0.5% BSA, 1 mM EDTA, 40 mM NaHPO₄ (pH 7.2), 5% SDS for washing at 60-65° C. One embodiment of medium stringency conditions includes hybridizing in 6 × SSC at about 45° C, followed by one or more washes in 0.2 × SSC, 0.1% SDS at 60° C. High stringency conditions include and encompass from at least about 31% v/v to at least about 50% v/v formamide and from about 0.01 M to about 0.15 M salt for hybridization at 42° C, and about 0.01 M to about 0.02 M salt for washing at 55° C. High stringency conditions also may include 1% BSA, 1 mM EDTA, 0.5 M NaHPO₄ (pH 7.2), 7% SDS for hybridization at 65° C, and (i) 0.2 × SSC, 0.1% SDS; or (ii) 0.5% BSA, 1 mM EDTA, 40 mM NaHPO₄ (pH 7.2), 1% SDS for washing at a temperature in excess of 65° C. One embodiment of high stringency conditions includes hybridizing in 6 × SSC at about 45° C, followed by one or more washes in 0.2 × SSC, 0.1% SDS at 65° C.

In certain embodiments, a corresponding Th1 immune status biomarker polynucleotide is one that hybridizes to a disclosed nucleotide sequence (e.g., SEQ ID NO: 3 or SEQ ID NO: 4) under very high stringency conditions. One embodiment of very high stringency conditions includes hybridizing 0.5 M sodium phosphate, 7% SDS at 65° C, followed by one or more washes at 0.2 × SSC, 1% SDS at 65° C.

Other stringency conditions are well known in the art and a skilled addressee will recognize that various factors can be manipulated to optimize the specificity of the hybridization. Optimization of the stringency of the final washes can serve to ensure a high degree of hybridization. For detailed examples, see Ausubel *et al., supra* at pages 2.10.1 to 2.10.16 and Sambrook *et al.* (1989, *supra*) at sections 1.101 to 1.104.

### 2.6.1 Biomarker detection

All the essential reagents required for detecting and quantifying the Th1 immune status biomarkers of the invention may be assembled together.

Reagents that allow quantification of a Th1 immune status biomarker include compounds or materials, or sets of compounds or materials, which allow quantification of the Th1 immune status biomarker. In specific embodiments, the compounds, materials or sets of compounds or materials permit determining the level or abundance of a polypeptide (i.e., a PD-L2 polypeptide).

The reagents described herein, which may be optionally associated with detectable labels, can be presented in the format of a microfluidics card, a chip or chamber, a microarray for use with the assays described in the examples or below, e.g., RT-PCR or Q PCR techniques described herein.

### 3. Diagnostic methods

The indicator can also be used for determining a likelihood of the subject having a disease that is associated with an undesirable Th1 immune response status. In this case, this would typically be achieved by comparing the indicator to at least one indicator reference, the indicator reference being indicative of the disease, and determining the likelihood in accordance with the results of the comparison. Non-limiting examples of Th1-related diseases include infectious diseases (particularly viral infections), autoimmune diseases, host versus graft disease (HVGD), tissue transplantation and proliferative disorders (*e*.*g*., a metastatic cancer).

In embodiments of this type, the at least one indicator reference is a distribution of indicators determined for a reference population. For example, if a subject presents with clinical symptoms of a pathogenic infection (*e*.*g*., hepatitis viruses, fungal infections such as aspergillus, human immunodeficiency virus (HIV), malaria, typhoid, cholera, herpes viruses, chlamydia, and HPV), then a reference group consisting of individuals with the same or a similar disease will be used to compare the indicator of the subject.

In some embodiments, a determination of the likelihood of a subject having the disease is made using more than one reference group of individuals. For example, a first reference group consisting of individuals previously diagnosed and known to have the disease of interest, and second reference group consisting of individuals diagnosed as having a healthy condition.

As described above, the methods of the present invention can be used to diagnose a disease that is associated with an elevated Th1 immune response and is therefore diagnosed when the level of PD-L2 and optionally PD-L1 in the sample obtained from the subject is above or below a predetermined threshold. Exemplary Th1-related diseases of this type are autoimmune diseases. For example, the autoimmune disease can be selected from any one of the group comprising: Alzheimer's disease, ankylosing spondylitis, atherosclerosis, autoimmune-associated infertility, autoimmune encephalomyelitis, autoimmune hemolytic anemia, autoimmune hemophilia, autoimmune hepatitis, autoimmune lymphoproliferative syndrome (ALPS), autoimmune thrombocytopenic purpura, autoimmune uveoretinitis, Barrett's esophagus, chronic fatigue syndrome (CFS/CFIDS/ME), bullous pemphigoid, chronic lyme disease (borreliosis), Crohn's disease, diabetes, depression, fibromyalgia (FM), gastritis, gastroesophageal reflux disease (GERD), glomerulonephritis (e.g., crescentic glomerulonephritis, proliferative glomerulonephritis), Goodpasture's syndrome, Grave's disease, Guillain-Barre syndrome, Hashimoto's thyroiditis, hemolytic anemia, hypertension, hyperthyroidism, hypothyroidism, idiopathic Addison's disease, insulin resistance, irritable bowel syndrome (IBS), interstitial cystitis (IC), kidney stones, Löfgren's syndrome, lupus erythematosus, mixed connective tissue disease, multiple chemical sensitivity (MCS), migraine headache, Morgellon's, multiple sclerosis, myasthenia gravis (MG), osteoarthritis, pemphigus (e.g, pemphigus vulgaris), pernicious anemia, polymyalgia rheumatic, polymyositis prostatitis, psoriasis, psoriatic arthritis, Raynaud's syndrome/phenomenon, reactive arthritis (Reiter syndrome), restless leg syndrome, reflex sympathetic dystrophy (RSD), rheumatoid arthritis, sarcoidosis, scleroderma, sinusitis, seasonal affective disorder (SAD), Sjögren's syndrome, ulcerative colitis, uveitis, and vertigo.

Suitably, the Th1-related disease may be an infection with a virus, bacteria, fungi, or parasite. Viruses include, but are not limited to, *Retroviridae* human immunodeficiency viruses, such as HIV -1 (also referred to as HTLV-III, LAV or HTLV-III/LAV, or HIV-III); and other isolates, such as HIV-LP); *Picornaviridae* (*e.g.,* polio viruses, hepatitis A virus; enteroviruses, human *Coxsackie* viruses, rhinoviruses, echoviruses); *Calciviridae* (*e.g.,* strains that cause gastroenteritis, including Norwalk and related viruses); *Togaviridae* (*e.g.,* equine encephalitis viruses, rubella viruses); *Flaviridae* (*e.g.,* dengue viruses, encephalitis viruses, yellow fever viruses); Coronoviridae (*e*.*g*., coronaviruses); Rhabdoviradae (*e*.*g*., vesicular stomatitis viruses, rabies viruses); *Filoviridae (e.g.,* ebola viruses); *Paramyxoviridae (e.g.,* parainfluenza viruses, mumps virus, measles virus, respiratory syncytial virus, metaneumovirus); *Orthomyxoviridae* (*e*.*g*., influenza viruses); *Bungaviridae (e.g.,* Hantaan viruses, bunga viruses, phleboviruses and Nairo viruses); *Arenavihdae* (hemorrhagic fever viruses); *Reoviridae* (*e*.*g*., reoviruses, orbiviurses and rotaviruses); *Bimaviridae; Hepadnaviridae* (Hepatitis B virus); *Parvovirida* (parvoviruses); *Papovaviridae* (papilloma viruses, polyoma viruses); Adenoviridae (most adenoviruses); *Herpesviridae* (herpes simplex virus (HSV) 1 and 2, varicella zoster virus, cytomegalovirus (CMV), herpes virus); *Poxyiridae* (variola viruses, VACV, pox viruses); and *Iridoviridae* (*e*.*g*., African swine fever virus); and unclassified viruses (*e*.*g*., the etiological agents of *Spongiform* encephalopathies, the agent of delta hepatitis (thought to be a defective satellite of hepatitis B virus), the agents of non-A, non-B hepatitis (class 1 = internally transmitted; class 2 = parenterally transmitted (i.e., Hepatitis C); and astroviruses.

In some embodiments, the pathogenic infection is a bacterial pathogen. Bacteria from which are known to be pathogenic in a subject include, but are not limited to, pathogenic *Pasteurella* species *(e.g., Pasteurella multocida),* Staphylococci species *(e.g., Staphylococcus aureus), Streptococcus* species *(e.g., Streptococcus pyogenes* (Group A *Streptococcus), Streptococcus agalactiae* (Group B *Streptococcus), Streptococcus* (viridans group), *Streptococcus faecalis, Streptococcus bovis, Streptococcus* (anaerobic sps.), *Streptococcus pneumoniae), Neisseria* species *(e.g., Neisseria gonorrhoeae, Neisseria meningitidis), Escherichia* species (e.g., enterotoxigenic *E. coli* (ETEC), enteropathogenic *E. coli* (EPEC), enterohemorrhagic *E. coli* (EHEC), and enteroinvasive *E. coli* (EIEC)), *Bordetella* species, *Campylobacter* species, *Legionella* species *(e.g., Legionella pneumophila), Pseudomonas* species, *Shigella* species, *Vibrio* species, *Yersinia* species, *Salmonella* species, *Haemophilus* species *(e.g., Haemophilus influenzae), Brucella* species, *Francisella* species, Bacterioides species, Clostridia species *(e.g., Clostridium difficile, Clostridium perfringens, Clostridium tetani), Mycobacteria* species *(e.g., M. tuberculosis, M. avium, M. intracellulare, M. kansaii, M. gordonae), Helicobacter pyloris, Borelia burgdorferi, Listeria monocytogenes, Chlamydia trachomatis, Enterococcus* species, *Bacillus anthracis, Corynebacterium diphtheriae, Erysipelothrix rhusiopathiae, Enterobacter aerogenes, Klebsiella pneumoniae, Fusobacterium nucleatum, Streptobacillus moniliformis, Treponema pallidium, Treponema pertenue, Leptospira, Rickettsia,* and *Actinomyces israeli.*

In other embodiments of the invention, the pathogenic infection is a eukaryotic pathogen, such as pathogenic fungi and parasites. Fungi that are known to be pathogenic at least to some extent include, but are not limited to, *Cryptococcus neoformans, Histoplasma capsulatum, Coccidioides immitis, Blastomyces dermatitidis, Candida albicans, Candida glabrata, Aspergillus fumigata, Aspergillus flavus,* and *Sporothrix schenckii.*

Other eukaryotic pathogens from which the heterologous antigen can be derived include, but are not limited to, pathogenic protozoa, helminths, *Plasmodium,* such as *Plasmodium falciparum, Plasmodium malariae, Plasmodium ovale,* and *Plasmodium vivax; Toxoplasma gondii; Trypanosoma brucei, Trypanosoma cruzi; Schistosoma haematobium, Schistosoma mansoni, Schistosoma japonicum; Leishmania donovani; Giardia intestinalis; Cryptosporidium parvum;* and the like.

Th1-related diseases also include any malignant or pre-malignant condition, proliferative or hyper-proliferative condition or any disease arising or deriving from or associated with a functional or other disturbance or abnormality in the proliferative capacity or behaviour of any cells or tissues of the body. Thus, the methods described herein could be used to diagnose a cancer, including assessing the likelihood whether a cancer is a metastatic cancer. For example, cancers which could be suitably diagnosed in accordance with the practices of this invention include breast cancer, colon cancer, lung cancer and prostate cancer, cancers of the blood and lymphatic systems (including Hodgkin's disease, leukemias, lymphomas, multiple myeloma, and Waldenstrom's disease), skin cancers (including malignant melanoma), cancers of the digestive tract (including head and neck cancers, esophageal cancer, stomach cancer, cancer of the pancreas, liver cancer, colon and rectal cancer, anal cancer), cancers of the genital and urinary systems (including kidney cancer, bladder cancer, testis cancer, prostate cancer), cancers in women (including breast cancer, ovarian cancer, gynecological cancers and choriocarcinoma) as well as in brain, bone carcinoid, nasopharyngeal, retroperitoneal, thyroid and soft tissue tumors.

The present invention also extends to the management of a Th1-related disease, or prevention of further progression of a Th1-related disease, or an assessment of the efficacy of therapies in subjects following positive diagnosis for the presence of a Th1-related disease, in a subject.

The present methods can be practiced in the field of predictive medicine for the purpose of diagnosis or monitoring the presence or development of a Th1-related disease in a subject, and/or monitoring response to therapy efficacy. The biomarker profiles and corresponding indicators further enable determination of endpoints in pharmacotranslational studies.

The above methods can be practiced not only to diagnose a Th-related disease, but also to identify whether a subject is responding or not responding to a treatment regimen. Using such methods, an assessment may be made relatively early in the treatment process, i.e., before clinical manifestations of efficacy. In this way, the treatment regimen can optionally be discontinued, a different treatment protocol can be implemented and/or supplemental therapy can be administered. Thus, in some embodiments, a sample Th1 immune status biomarker profile is obtained within about 2 hours, 4 hours, 6 hours, 12 hours, 1 day, 2 days, 3 days, 4 days, 5 days, 1 week, 2 weeks, 3 weeks, 4 weeks, 6 weeks, 8 weeks, 10 weeks, 12 weeks, 4 months, six months or longer of commencing therapy.

The method of the invention is implemented using one or more processing devices. In some embodiments, these methods comprise: (1) determining a pair of biomarker values, the pair of biomarker values being selected from the group consisting of a pair of biomarker values indicative of a concentration of PD-L2 polypeptide and PD-L1 polypeptide; (2) determining an indicator indicative of a ratio of the concentrations of the polypeptides using the pair of biomarker values; (3) retrieving previously determined indicator references from a database, the indicator reference(s) being determined based on indicators determined from at least one group of a reference population, the at least one group comprises individuals diagnosed with a Th1-related disease; (4) comparing the indicator to the indicator reference(s); (5) using the results of the comparison to determine a probability indicative of the subject having or not having the Th1-related disease; and (6) generating a representation of the probability, the representation being displayed to a user to allow the user to assess the likelihood of a subject having the Th1-related disease.

Additionally, the present invention encompasses methods for differentiating between metastatic cancer and non-metastatic cancer (e.g., benign lesions) in a subject. These methods suitably comprise: (a) obtaining a sample taken from a subject showing a clinical sign of metastatic cancer or non-metastatic cancer, the sample comprising cells that interact with IEC (e.g., APCs such as dendritic cells, or tumor cells); (b) determining a Th1 immune status biomarker profile of the sample, wherein the Th1 immune status biomarker profile comprises a biomarker value for at least one (e.g., 2, 3, 4, 5, 6, 7, 8, 9, 10, or more) Th1 immune status biomarker, wherein the biomarker value is at least partially indicative of a concentration of the Th1 immune status biomarker, and wherein the at least one Th1 immune status biomarker comprises PD-L2, and optionally PD-L1, of IEC-interacting cells (e.g., APCs or tumor cells) in the sample; (c) determining an indicator by: determining an indicator that is indicative of the concentration of the at least one Th1 immune status biomarker; (d) retrieving previously determined indicator references from a database, wherein the indicator references are distributions of indicators determined for groups of a reference population, the first group consisting of individuals diagnosed with metastatic cancer, and the second group consisting of individuals diagnosed with non-metastatic cancer (*e*.*g*., benign lesions); (e) comparing the indicator to the indicator references; (g) using the results of the comparison to determine a probability of the subject being classified within the first or second group; (f) generating a representation at least partially indicative of the indicator and the probability; and (g) providing the representation to a user to allow the user to assess the likelihood of a subject having metastatic cancer or non-metastatic cancer.

In order that the invention may be readily understood and put into practical effect, particular preferred embodiments will now be described by way of the following non-limiting examples.

### EXAMPLES

### EXAMPLE 1

### PD-L2 EXPRESSION OF DCs INVERSELY CORRELATES WITH MALARIA SEVERITY IN HUMANS

To determine if PD-L1 and PD-L2 influenced malarial immunity, seven malaria-naive, healthy human volunteers were infected with 1800 *P. falciparum* infected red blood cells (pRBC) and their blood examined before and seven days after challenge. We examined DCs, defined by CD11c expression, in view of their important role in pathogenesis (Wykes and Good, 2008) and since PD-L1 and PD-L2 on DCs can down-regulate immune responses by T cells (Brown et al., J. Immunol. 170: 1257-1266, 2003; Freeman et al., J. Exp. Med. 192: 1027-1034,2000). In all seven volunteers, 90% of DCs expressed PD-L1 before infection, and there was no significant change in the percentage of DCs expressing this ligand by day seven of infection (Figure 1A). In contrast, while 80% of DCs also expressed PD-L2 before infection, five of seven individuals showed a significantly reduced (17- 57%) percentage of PD-L2⁺ DCs at day seven post infection (Figure 1B). Notably, significant inverse correlation was observed between the level of parasitemia and the ratio of percentage PD-L2 to PD-L1 expression on DCs at day seven post infection (Figure 1C). Overall, contrary to the generally perceived role of PD-L2 as an immune inhibitor, higher frequencies of PD-L2- expressing DCs were associated was observed in individuals with lower parasitemia after infection with *P. falciparum.*

### Materials and Methods

### Human studies

The method for conduct of the clinical trial (McCarthy et al., PLoS One. 6: e21914, 2011) (ClinicalTrials.gov identifier: NCT02389348) and the PCR method used to quantify parasitemia (Rockett et al., Malar. J. 10: 48, 2011) are described in detail elsewhere. Each participant gave informed consent. Seven of eight healthy volunteers (n = 4 males; and n = 3 females) aged 19 - 55 years (median age, 24 years {interquartile range, 21- 37}) who participated in a study to evaluate the effectiveness of the experimental anti-malarial therapeutics OZ439 and DSM265 separately consented to participate in this sub-study, nested within the clinical trial. This study was approved by the Human Research Ethics Committee of the QIMR Berghofer Institute for Medical Research (QIMR). Volunteers received approximately 1800 *P. falciparum* pRBCs *via* intravenous injection in 2.0 mL of saline. On day seven, the day designated for commencement of treatment, participants were admitted to the study unit and administered the investigational anti-malarial drug treatment after blood was collected for the study.

### EXAMPLE 2

### PD-L2 EXPRESSION ON DCs INVERSELY CORRELATES WITH MALARIA SEVERITY IN MICE

To understand the biological relevance of these data, the present inventors next investigated four mouse models of malaria. They chose four different species/strains of *Plasmodium* that infect mice, with each showing distinct biology and pathogenicity. When WT mice were infected with non-lethal *P. yoelii* 17XNL or *P. chabaudi,* and the blood was examined every 1 - 3 days for parasites, the infection progressed at different rates, but both groups cleared the infection within ~ 30 days (Figure 2A). In contrast, WT mice infected with *P. yoelii* YM or *P. berghei* ANKA showed severe, but distinct disease courses (Figure 2B; monitored as per Table 1 and 2). *P*. *berghei* parasitemia is low compared to *P. yoelii* YM infections because *P.* berghei-infected RBC sequester from the blood into deep tissues including the brain, leading to lethal cerebral disease. However, all *P. yoelii* YM and *P*. *berghei-infected* mice had to be euthanized within 10 days when the clinical score was ≥ 4 (Table 1 and 2).

The present inventors examined surface expression of PD-L1 and PD-L2 on DCs from the spleen, which has been shown to be a major site of parasite killing and regulation of parasite-specific immune responses in mice (Yadava et al., Proc. Natl. Acad. Sci. USA, 93: 4595-4599, 1996). Approximately 70% of CD11c⁺ DCs in the spleens of native mice expressed PDL1 and this percentage increased in *P. berghei* and *P. chabaudi-infected* mice but not during lethal or non-lethal *P. yoelii* infections (Figure 3A). PD-L1-expressing DCs did show increases in the level of surface expression (MFI) of PD-L1I following all four malarial infections compared to DCs from native mice, with non-lethal *P. chabaudi* -infected mice showing the greatest increase (Figures 3B and 4A-F). In contrast, < 5% of splenic DCs from native mice expressed PD-L2. This differed from human blood DCs that predominantly expressed PD-L2, which most likely reflects their different origins from blood and spleen. Furthermore, the percentages of PD-L2⁺ DCs increased during all malarial infections with significantly greater percentages found in mice with non-lethal than lethal malaria (Figures 3C and 4A-E). The MFI of PD-L2 staining on PD-L2-expressing DCs also increased in mice infected with all but *P. berghei* parasites, compared to DCs from native mice (Figures 3D and 4A-F).

Finally, CD11c⁺ DCs from lethal and non-lethal *P. yoelii* malaria showed similar increases in PD-L1 and PD-L2 mRNA levels (Figure 4G) suggesting that the difference in PD-L2 between these parasites noted in Figure 3C is dependent on post-transcriptional regulation or protein localization. Of note, DCs from lethal and non-lethal *P. yoelii* infections had the same surface levels of PD-L1 and PD-L2 expression and mRNA but differed in percentages of PD-L2⁺ and not PD-L1⁺ DCs.

Overall, the results from all infections are consistent with a hypothesis that a higher percentage of PD-L2⁺ DCs correlates with a favourable disease outcome.

### Materials and Methods

### Mice studies

Specific pathogen-free C57BL/6J (wt) female mice 8-12 weeks of age were obtained from the Animal Resources Centre (Perth, Australia). Mice were housed in the QIMR animal research facility, and all procedures approved and monitored by the QIMR Animal Ethics Committee. Work was conducted under QIMR animal ethics approval number A0209-622M in accordance with the "Australian code of practice for the care and use of animals for scientific purposes" (Australian National Health & Medical Research Council). PD-1 knockout (ko) *(Pdcd1*^{*-*/}*⁻)* mice on a C57BL/6 background were kindly provided by Dr. T. Honjo through the Riken BRC (Nishimura et al., Science. 291: 319-322, 2001). The PD-L2 ko (Liang et al., Eur. J. Immunol. 36: 58-64, 2006), PD-L1 ko (Liang et al., Eur. J. Immunol. 36: 58-64, 2006) and PD-1 ko mice on a C57BL/6J background, used in these studies, were confirmed to have the gene deleted by PCR testing and/or flow cytometry. The sample size was estimated based on previous studies with similar assays, using the same parasites.

For experiments with multiple groups, all mice were first infected and then randomly assigned into treatment groups. No blinding was undertaken.

### Parasitic infection and monitoring

Cohorts of 3-6 WT mice were infected intravenously with 10⁵ *P. yoelii* 17XNL, 10⁵ *P. chabaudi* AS, 10⁴ *P. yoelii* YM, or 10⁴ *P. berghei* ANKA parasitized red blood cells (pRBCs) freshly obtained from C57BL/6J mice previously infected mice. These parasite doses were previously shown to give obvious parasitemia around the same time. Tail-tip blood films were made every 1-2 days, stained using the Quick Dip modified Wright-Giemsa stain (Thermo Fisher Scientific) and examined for parasitemia, for up to 60 days. The percentage of pRBCs was assessed by counting at least 300 RBCs during parasitemia > 1% and 20 fields with around 10,000 cells at other times.

The mean percentage parasitemia shown in several figures is the mean percentage pRBC of total RBC, from individual mice in a group. Mice were monitored daily for anemia, and physical symptoms of disease, including posture (hunching), lack of activity and fur texture. Mice were euthanized if they showed signs of significant distress as described in Tables 1 and 2, below.

**TABLE 1**

| **Criteria** | **Grade 0** | **Grade 1** | **Grade 2** |
|---|---|---|---|
| Weight loss | < 10% | 10 to 25% | |
| Posture | Normal | Hunching noted only at rest | Severe hunching impairs movement |
| Activity | Normal | Mild to moderately decreased | Stationary unless stimulated |
| Fur texture | Normal | Mild to moderate ruffling | Sever ruffling/poor grooming |
| Hemoglobin | Normal | < 50 g/L | < 20 g/L |

*P. yoelii* YM symptoms include anemia respiratory distress, and haematuria with complications such as coma and convulsions but never cerebral malaria. The mice are monitored daily by the above criteria for distress during the period of the experiment, to determine whether treatments described in the study are causing distress to mice to a degree to where they should be euthanized. If the cumulative score reaches above 3 by these assessment criteria, or if the weight loss is more than 25%, the distressed mouse is euthanized.

**TABLE 2**

| **Symptoms** | **Score** | **Day post-infection** |
|---|---|---|
| Ruffled fur | *1* | 5 |
| Hunching | *1* | 5 |
| *Wobbly gait* | *1* | *6* |
| *Limb paralysis* | *1* | *6* |
| *Convulsions* | *1* | *6-7* |
| *Coma* | *1* | *6-7* |

*P. berghei* causes lethal cerebral disease and symptoms are usually evident by day 7 post infection. Scores are cumulative and mice with a cumulative score = 4 are euthanized. Notably, ruffled fur and hunching are general clinical sigs, while other symptoms (in italics) are symptoms of cerebral malaria.

### Flow cytometry

Single-cell suspensions of processed blood or spleen cells were labeled with combinations of fluorophore-conjugated antibodies shown below. Fixable Viability Dye eFluor780 (eBioscience) was used to exclude dead cells from analysis. Serial dilutions of each antibody were pre-tested by flow cytometry to determine the optimal concentration for the main assay. Anti-CD16/32 (clone 2.4G2, BD) was used for blocking nonspecific Fc binding. Intracellular markers denoted with an asterisk were labeled following fixation and permeabilization of cells using BD Pharmingen Transcription Factor Buffer Set. Acquisition of data was performed using a BD LSR Fortessa flow cytometer and BD FACSDiva software. Analysis of data was performed using FCS express (De Novo Software) or FlowJo (Tree Star).

**TABLE 3**

| **Marker** | **Antibody done** | **Conjugate** | **Company** |
|---|---|---|---|
| CD11c | N418 | SV421 | Biolegend |
| | 3.9 | BV-605 | Biolegend |
| PD-L1 | 10F.9G2 | PE | BD |
| | 29E.2A3 | PE-Cy7 | Biolegend |
| PD-L2 | TY25 | APC | BD |
| | 24F.10C12 | Alexa Fluor | Biolegend |
| | | 647 | |
| PD-1 | RMP1-14 | PE-Cy7 | BioXell |
| | J43 | | eBioscience |
| CD4 | GK1.5 | Pacific Blue | Biolegend |
| CD62L | MEL-14 | BV605 | Biolegend |

### EXAMPLE 3

### PD-L2 is REQUIRED/OR SURVIVAL AND PARASITE CONTROL

To determine the contribution of PD-L2 to the control of malarial parasites, the present inventors next examined the outcome of *P. yoelii* 17XNL infection in PD-L2 ko mice (Liang et al., Eur. J. Immunol. 36: 58-64, 2006) (on a C57BL/6J background) compared with wt mice. All wt mice cleared the infection within 27 days (Figure 5A).

However, the PD-L2 ko mice had significantly higher parasitemia than wt mice after day 13, and all of these mice died or had to be euthanized by day 19 (Figure 5A and Figure 6A) due to clinical scores ≥ 4 (Figure 6B). Thus, PD-L2 expression is required for parasite control and survival from infection with *P. yoelii* 17XNL.

To confirm our observation that PD-L2 was required to survive *P. yoelii* 17XNL infections, we next blocked PD-L2 with a monoclonal antibody when parasites became detectable in the blood. For this experiment, wt mice were infected with *P. yoelii* 17XNL and given either anti-PD-L2 or control rat IgG, four days post infection and every 3-4 days until day 14-18 post infection. All wt mice that received rat IgG survived and cleared the infection within 32 days (Figure 5B and Figure 6C). In contrast, 100% of the infected mice that were given the PD-L2 blocking antibody died, or were euthanized, by day 19, due to severe symptoms (Figure 6D), although the degree of parasite control was similar in anti-PD-L2 and control antibody treated groups (Figure 5B and Figure 6C). This was in contrast to PD-L2 ko mice, which had significantly higher parasitemia after day 13 (Figure 5A) suggesting either that the antibody did not completely inhibit function, or that four days of PD-L2 function, before blockade, partially improved immunity.

To further explore the role of PD-L2 in protection against another non-lethal infection, wt mice were infected with non-lethal *P. chabaudi* malaria and treated with either anti-PD-L2 or rat IgG (Figure 5C and Figure 6E) as for *P. yoelii* 17XNL experiments. Mice from both groups survived but blockade of PD-L2 significantly increased parasitemia during the acute infection (day 8; note log scale), led to generally higher parasitemia during the chronic phase of infection (> day 21) and delayed parasite clearance by four days (arrow indicates parasite clearance in rat IgG-treated mice; Figure 5C). Overall, these protection/survival studies showed that PD-L2 expression was required for better control of non-lethal malarias and survival from P. *yoelii* 17XNL malaria.

### EXAMPLE 4

### PD-L2 IMPROVES PARASITE-SPECIFIC CD4⁺ T CELL RESPONSES IN MICE

We next focused on understanding why mice did not survive infection with non-lethal *P. yoelii* 17XNL when PD-L2 was blocked. We therefore repeated the above blocking experiments and collected spleens at days 7 and 14 for evaluation by multiple immunoassays. First, CD4⁺ T cells were examined for the expression of T_{bet}, a transcription factor required for effector functions of Th1 CD4⁺ T cells, which are known to mediate protection against malaria (Kumar and Miller, Immunol Lett, 25: 109-114,1990; Stephens and Langhorne, PLoS Pathog, 6: e1001208, 2010; and Su and Stevenson, J Immunol, 168: 1348-1355, 2002). T cells were also evaluated for expression of CD62L, a marker found on native T cells and which also distinguishes central memory (CD62L^{hi}) from effector memory (CD62L^{lo}) T cells (Figure 7A). Compared to native mice (day 0; Figure 8A), there was a significant increase in numbers of T_{bet}-expressing CD62L^{hi} CD4⁺ T cells per spleen by day 7 (Figure 8B; p < 0.0095) in control mice given Rat IgG but not mice with PD-L2 blockade (Figure 8B; p > 0.05). By day 14, the control mice had 2.2 and 3-fold more T_{bet}-expressing CD62L^{hi} and CD62L^{lo} CD4⁺ T cells per spleen, respectively, than the mice given anti-PD-L2 antibody (Figure 8C). Similarly, control mice had > five-fold higher numbers of IFN-γ-secreting, parasite-specific CD4⁺ T cells at day 14 as measured by responses to parasite antigen MSP1₁₉ in culture, than mice with PD-L2 blockade (Figure 8D). An in vitro EdU-uptake assay confirmed that control mice had higher numbers of parasite-specific CD4⁺ T cells which proliferated in response to parasite antigen (Figure 8E). However, levels of serum IFN-γ were not significantly affected by PD-L2 blockade (Figure 8F). In contrast, mice with PD-L2 blockade had greater than two-fold more serum IL-10 than control mice by day 14 (Figure 8G). This result correlated with a significant increase in numbers of regulatory T cells (T_{reg}) per spleen seen with PD-L2 blockade compared to control treated mice (Figure 8H).

Studies with *P. yoelii* 17XNL-infected PD-L2 ko mice also found significantly lower numbers of T_{bet} expressing and IFN-γ-secreting, parasite-specific CD4⁺ T cells per spleen at day 14 compared to infected WT mice (Figures 7B and C). Finally, there was no significant reduction in IFN-γ-secreting, parasite-specific CD8⁺ T cells per spleen at day 14 in infected PD-L2 ko mice or infected mice given anti-PD-L2 blocking antibody compared to infected wt mice (Figure 7DI).

Overall, the data presented herein show that PD-L2 expression is necessary for effective Th1 CD4⁺ T cell responses against *P. yoelii* 17XNL malaria. Given that a higher ratio of PD-L2 to PD-L1 expression on DCs was associated with lower parasitemia and blockade of PD-L2 resulted in reduced Th1 responses, we hypothesized PD-L2 may inhibit PD-L1 functions which were reported to inhibit Th1 responses (Liang et al., Eur. J. Immunol. 36: 58-64, 2006). Furthermore, PD-L2 blockade caused mortality in mice infected with *P. yoelii* 17XNL but not *P. chabaudi* malaria. In alignment, PD-L1/PD-1-mediated immune suppression was previously shown to be greater during the acute phase of *P. yoelii* 17XNL (Butler et al., Nat. Immunol. 13: 188-195, 2012) than *P. chabaudi* malaria (Horne-Debets et al., Cell. Reports. 5: 1204-1213, 2013). As such, we concluded that PD-L2-mediated inhibition of PD-L1/PD-1-mediated immune suppression, could explain the different outcomes of PD-L2 blockade between the two infections.

### EXAMPLE 5

### PD-L1 AND PD-L2 CO-EXPRESSION ON DCs DETERMINE IMMUNITY

The present inventors next undertook DC-transfer studies to establish if PD-L1 expression on DCs was responsible for lethality of malaria. To do so, wt and PD-L1 ko mice were infected with lethal *P. yoelii* YM malaria, DCs isolated at day 7 post infection (Figures 9A and eB) and transferred to native mice which were then infected with lethal *P. yoelii* YM malaria. While 100% mice given DCs from wt mice had to be euthanized within ten days due to clinical scores ≥ 4, all mice given DCs from PD-L1 ko mice survived (Figure 9C) and cleared the infection (Figures 9D and 9E). This transfer study showed that PD-L1 on DCs was mediating lethality as mice given DCs with abundant PD-L1 but little PD-L2 (see, Figures 3A, C and Figure 4D) did not survive, while ail mice given PD-L1 ko DCs survived.

WT and PD-1 ko mice were then infected with lethal *P. yoelii* YM to confirm that the PD-1 pathway was responsible for the lethality of *P. yoelii* YM malaria. While 100% of WT had to be euthanized by day 10 due to clinical scores ≥ 4, all PD-1 ko mice survived (Figure 9F) and cleared the infection (Figures 9G and 9H) confirming that the PD-1/PD-L1 pathway was driving lethality of *P. yoelii* YM infections. Overall, these studies showed PD-1 and PD-L1 mediated lethality of malaria.

Given that PD-L2 expression was associated with survival from malaria, the present inventors next examined how PD-L2 co-expression with PD-L1 on DCs could modulate immunity. A previous study showed that the interaction between PD-L1 on DCs and PD-1 on CD8⁺ OTI T cells contributed to ligand-induced T cell receptor (TCR) down-modulation (Karwacz et al., EMBO. Mol. Med. 3: 581-592, 2011). It was therefore decided to investigate if PD-L2 co-expression with PD-L1 on DCs could inhibit PD-L1-mediated down-regulation of TCRs and inducible T-cell costimulator (ICOS) expression. To do so, purified DCs and T cells were cultured from infected mice (1:5 cells), with antibodies to block PD-1, PD-L1 or PD-L2 functions and examined the T cells after 36 hours for high expression of CD3, a component of the TCR, and high ICOS expression which can indicate T cell activation (Figures 4I-4N). Blockade of PD-1-signalling to T cells with anti-PD-1 antibody in the DC: T cell cultures significantly increased the expression of CD3 and ICOS, indicating PD-1 signals down-regulated expression of these molecules on T cells (Figures 4K and 4N). When PD-L1 signals were blocked with antibody, leaving only PD-L2 to function, T cells had significantly increased levels of ICOS and CD3 (Figures 4L and 4N). In contrast, when PD-L2 was blocked, leaving PD-L1 function intact, there was a significant loss of CD3 and ICOS (Figures 4M and 4N). Overall, these findings show that in the context of cells from *P. yoelii 17XNL-*infected mice, PD-L1 expression on DCs is likely to inhibit T cell activation, whilst PD-L2 appears to promote CD3 and ICOS expression.

### Materials and Methods

### DC transfer study

CD11c⁺ DC obtained from spleens of wt and PD-L 1 ko mice infected with 10⁴ *P. yoelii* YM (lethal) pRBC. Four days post infection, mice were treated with 250 µg Pyrimethamine daily for four days to clear the infection. At day 7, the spleens were digested and DC enriched using Dynal DC enrichment kit. Samples were run on the AutoMACs to remove residual Dynal labeled cells and hemozoin. Highly purified DCs were obtained by labelling DCs with anti-CD11c MACS beads and isolated on AutoMACS. Approximately 1.5 x 10⁷ DC were then transfused intravenously to naive mice . After resting the mice for greater than 15 hours, they were infected with a lethal dose of *P. yoelii* YM (10⁴ pRBC). Mice were followed for 48 days when monitoring was stopped.

### DC-T cell culture

Mice were infected with 10⁵ *P. yoelii* 17XNL pRBC and on day 14 post infection, the spleens were digested and total T cells were isolated using CD90.2 MACS beads, to minimize any effect on the TCR. DCs were then isolated from remaining spleen cells using Dynal DC enrichment kit.

Approximately 10⁶ T cells were cultured with 2 x 10⁵ DCs in at least triplicate wells. Control or blocking anti-PD-1 (RMP1-14), anti-PD-L1 (10F.9G2) or anti-PD-L2 (TY25) antibodies were added to cultures at a concentration of 20 µg/ml. After 36 hours culture, cells were washed and labeled for flow cytometry. CD3 and ICOS expression was assessed on viable CD4⁺CD62L^{lo}PD-1⁺ T cells.

### EXAMPLE 6

### PD-L2 EXPRESSION ON DCs FROM PATIENTS WITH METASTATIC CANCER

To provide additional proof of concept data, blood DCs from patients with either benign lesions or metastatic melanoma were compared. A significant loss of PD-L2⁺ DCs was observed in patients suffering from metastatic disease (see, Figure 10). While healthy volunteers have a ratio of %PD-L2:%PD-L1 of around 0.9, this ratio drops to between 0.4 to 0.8 during metastatic melanoma. Interestingly, in patients with localized lesions (i.e., benign tumors), the %PD-L2:%PD-L1 ratio increases to between 0.9 and 1.3.

Based on the findings with systemic malaria and cancer, it appears that PD-L2, but not PD-L1 predicts the severity of Th1 immune response-associated systemic disease. Overall, it is predicted that PD-L2 levels increase during autoimmune disease, which drives expansion of damaging effector cells. This is reflected by patients with local lesions having a higher ratio.

### EXAMPLE 7

### PD-L2 MULTIMERIZATION IS INDICATIVE OF TH1 IMMUNE STATUS

The present inventors hypothesized that multimeric soluble PD-L2 (sPD-L2) would outcompete PD-L1 for binding to PD-1 on Th1 cells and thus reduce the suppressive effects of PD-L1 on T cell functions. To test this, wild-type mice were infected with lethal *P. yoelii* YM or *P. berghei* and administered PD-L2 on day 3, after parasitemia(s) were measurable and then on days 5 and 7 post-infection. All wild-type mice infected with *P. yoelii* YM and treated with control human IgG (Control Ig) died or had to be euthanized within ten days (Figure 11A-C).

Similarly, dimeric PD-L2 did not offer any protection from increasing parasitemia (Figure 11D). In contrast, 92% of P. *yoelii* YM-infected mice (n = 12) treated with PD-L2 survived and cleared the infection in 25 days with fewer symptoms (Figure 11A-E). All of the surviving mice were rested until day 150 and re-challenged with the same dose of lethal *P. yoelii* YM malaria (no additional PD-L2 was administered; Figure 11A) along with new age-matched, naïve control mice (Control Ig-R). All of the mice previously treated with PD-L2 survived re-infection with no symptoms, and only 4 out of 8 mice showed any parasitemia, as shown by a log scale in the Figure 11B. Within 20 days of re-infection, 80% of these sPD-L2-treated, re-infected mice had completely cleared the infection, as the transfer of 200 µl of blood from these mice to naïve mice did not transfer the infection (Figure 11F). In comparison, the second set of age-matched control mice succumbed to the infection, confirming the lethality of the parasite used for re-infections (Figure 116C). Overall, multimeric PD-L2 could overcome PD-L1 mediated lethality following infection with *P. yoelii* YM.

Similarly, 100% of control mice infected with *P. berghei* developed experimental cerebral malaria symptoms (ECM) within 8 days (Figure 11G) and succumbed to the infection by day 10 (Figure 11H). Only 22% of the P. berghei-infected mice treated with sPD-L2 developed cerebral malaria as seen by their ECM scores (Figure 11G). Furthermore, the surviving mice controlled the infection for approximately 20 days (Figure 11H and I), before succumbing 13 days after the last dose of PD-L2. Additional doses did not improve survival (data not shown). In summary, the administration of multimeric PD-L2 significantly improved survival from lethal infections and reduced the severity of the clinical symptoms, especially for cerebral malaria.

### EXAMPLE 8

### PD-L2 EXPRESSION ON BLOOD DCs IS INCREASED IN PATIENTS WITH IBD BUT NOT AFTER TNF BLOCKADE

Approximately 15 mL of blood collected was in lithium heparin from healthy human volunteers and patients diagnosed with Crohns Disease (CD; 12 samples) and 4 Ulcerative colitis patients (UC; 4 samples) and 7 IBD patients after treatment with TNF blockade 7 samples). PBMCs were isolated from blood following layering on Ficoll Paque Plus (GE Health, USA) and centrifugation as recommended by the manufacturer. The buoyant cells, free of red cells and granulocytes, were labelled with a DC lineage negative kit (Becton Dickinson, US; labels all cells except DC), CD11c, HLA-DR, PD-L1 and PD-L2. This will separate all lineage positive cells (CD3⁺, CD4⁺, CD8⁺, CD19⁺, CD56⁺, CD16⁺ and CD14⁺) from CD11c⁺ DC. The DCs from these individuals were examined for changes to PD-L1 and PD-L2 expression by flow cytometry. The GMI of PD-L1 and PD-L2 expression on Lineage-/MHC Class II+/CD11c+ DC were calculated.

To determine whether PD-L2 had clinical relevance to IBD patients, PD-L1 and PD-L2 expression on blood DCs from patients with CD and UC, CD/ UC patients following TNF blockade (aTNF) and Red Cross blood donors (RC; Figure 1) were examined. Geometric mean florescence intensity (GMI) of PD-L1⁺ and PD-L2⁺ DCs were assessed using flow cytometry. The GMIs of PD-L1 on DCs from UC and CD patients were similar to RC donors while TNF blockade showed a significant reduction of this immunosuppressive molecule (Figure 12A). In contrast, while the GMI of PD-L2⁺ DCs were generally low in naive donors, they were increased and similar in both CD and UC patients (Figure 12B). In contrast, anti-TNF-treated patients did not show the same increase in PD-L2. When the ratio of PD-L2: PD-L1 was calculated, to take into account changes in expression of both ligands in each individual, UC patients had a higher ratio of PD-L2: PD-L1 GMI on DCs than CD but both were indicating inflammation compared to controls (Figure 12C). The patients with TNF blockade did not show this increase. This assay was thus able to reflect responses to TNF-blockade therapy. These studies indicated that measuring the ratios of PD-L2: PD-L1 expression on blood DCs in patients with IBD reflected inflammation in the gastrointestinal tract

**TABLE 4**

| **SEQ ID NO:** | **Sequence Accession No.)** | **Sequence** |
|---|---|---|
| **1** | Human PD-L2 polypeptide (Q9BQ51) | MIFLLLMLSLELQLHQIAALFTVTVPKELYIIEHGSNVTLECNFDTGSHV NLGAITASLQKVENDTSPHRERATLLEEQLPLGKASFHIPQVQVRDEG QYQCIIIYGVAWDYKYLTLKVKASYRKINTHILKVPETDEVELTCQATG YPLAEVSWPNVSVPANTSHSRTPEGLYQVTSVLRLKPPPGRNFSCVF WNTHVRELTLASIDLQSQMEPRTHPTWLLHIFIPFCIIAFIFIATVIALR KQLCQKLYSSKDTTKRPVTTTKREVNSAI |
| **2** | Human PD-L1 polypeptide (Q9NZQ7) | MRIFAVFIFMTYWHLLNAFTVTVPKDLYVVEYGSNMTIECKFPVEKQL DLAALIVYWEMEDKNIIQFVHGEEDLKVQHSSYRQRARLLKDQLSLG NAALQITDVKLQDAGVYRCMISYGGADYKRITVKVNAPYNKINQRILV VDPVTSEHELTCQAEGYPKAEVIWTSSDHQVLSGKTTTTNSKREEKLF NVTSTLRINTTTNEIFYCTFRRLDPEENHTAELVIPELPLAHPPNERTHL VILGAILLCLGVALTFIFRLRKGRMMDVKKCGIQDTNSKKQSDTHLEE T |
| **3** | Human PD-L2 gene (AF329193) | ACGCGGGGTTTTTCTTCTCTTGAATATATCTTAACGCCAAATTTTGA GTGCTTTTTTTGTTACCCATCCTCATATGTCCCAGCTAGAAAGAATC CTGGGTTGGAGCTACTGCATGTTGATTGTTTTGTTTTTCCTTTTGGC TGTTCATTTTGGTGGCTACTATAAGGAAATCTAACACAAACAGCAAC TGTTTTTTGTTGTTTACTTTTGCATCTTTACTTGTGGAGCTGTGGCA |
| | | AGTCCTCATATCAAATACAGAACATGATCTTCCTCCTGCTAATGTTG AGCCTGGAATTGCAGCTTCACCAGATAGCAGCTTTATTCACAGTGA CAGTCCCTAAGGAACTGTACATAATAGAGCATGGCAGCAATGTGAC CCTGGAATGCAACTTTGACACTGGAAGTCATGTGAACCTTGGAGCA ATAACAGCCAGTTTGCAAAAGGTGGAAAATGATACATCCCCACACC GTGAAAGAGCCACTTTGCTGGAGGAGCAGCTGCCCCTAGGGAAGG CCTCGTTCCACATACCTCAAGTCCAAGTGAGGGACGAAGGACAGTA CCAATGCATAATCATCTATGGGGTCGCCTGGGACTACAAGTACCTG ACTCTGAAAGTCAAAGCTTCCTACAGGAAAATAAACACTCACATCCT AAAGGTTCCAGAAACAGATGAGGTAGAGCTCACCTGCCAGGCTAC AGGTTATCCTCTGGCAGAAGTATCCTGGCCAAACGTCAGCGTTCCT GCCAACACCAGCCACTCCAGGACCCCTGAAGGCCTCTACCAGGTC ACCAGTGTTCTGCGCCTAAAGCCACCCCCTGGCAGAAACTTCAGCT GTGTGTTCTGGAATACTCACGTGAGGGAACTTACTTTGGCCAGCAT TGACCTTCAAAGTCAGATGGAACCCAGGACCCATCCAACTTGGCTG CTTCACATTTTCATCCCCTCCTGCATCATTGCTTTCATTTTCATAGCC ACAGTGATAGCCCTAAGAAAACAACTCTGTCAAAAGCTGTATTCTTC AAAAGACACAACAAAAAGACCTGTCACCACAACAAAGAGGGAAGTG AACAGTGCTATCTGAACCTGTGGTCTTGGGAGCCAGGGTGACCTG ATATGACATCTAAAGAAGCTTCTGGACTCTGAACAAGAATTCGGTG GCCTGCAGAGCTTGCCATTTGCACTTTTCAAATGCCTTTGGATGAC CCAGCACTTTAATCTGAAACCTGCAACAAGACTAGCCAACACCTGG CCATGAAACTTGCCCCTTCACTGATCTGGACTCACCTCTGGAGCCT ATGGCTTTAAGCAAGCACTACTGCACTTTACAGAATTACCCCACTG GATCCTGGACCCACAGAATTCCTTCAGGATCCTTCTTGCTGCCAGA CTGAAAGCAAAAGGAATTATTTCCCCTCAAGTTTTCTAAGTGATTTC CAAAAGCAGAGGTGTGTGGAAATTTCCAGTAACAGAAACAGATGG GTTGCAATAGAGTTATTTTTTATCTATAGCTTCCTCTGGG |
| **4** | Human PD-L1 gene (AF177937) | ATGAGGATATTTGCTGTCTTTATATTCATGACCTACTGGCATTTGCT GAACGCATTTACTGTCACGGTTCCCAAGGACCTATATGTGGTAGAG TATGGTAGCAATATGACAATTGAATGCAAATTCCCAGTAGAAAAAC AATTAGACCTGGCTGCACTAATTGTCTATTGGGAAATGGAGGATAA GAACATTATTCAATTTGTGCATGGAGAGGAAGACCTGAAGGTTCAG CATAGTAGCTACAGACAGAGGGCCCGGCTGTTGAAGGACCAGCTC TCCCTGGGAAATGCTGCACTTCAGATCACAGATGTGAAATTGCAGG ATGCAGGGGTGTACCGCTGCATGATCAGCTATGGTGGTGCCGACT ACAAGCGAATTACTGTGAAAGTCAATGCCCCATACAACAAAATCAA CCAAAGAATTTTGGTTGTGGATCCAGTCACCTCTGAACATGAACTG ACATGTCAGGCTGAGGGCTACCCCAAGGCCGAAGTCATCTGGACA AGCAGTGACCATCAAGTCCTGAGTGGTAAGACCACCACCACCAATT CCAAGAGAGAGGAGAAGCTTTTCAATGTGACCAGCACACTGAGAAT CAACACAACAACTAATGAGATTTTCTACTGCACTTTTAGGAGATTAG ATCCTGAGGAAAACCATACAGCTGAATTGGTCATCCCAGAACTACC TCTGGCACATCCTCCAAATGAAAGGACTCACTTGGTAATTCTGGGA GCCATCTTATTATGCCTTGGTGTAGCACTGACATTCATCTTCCGTTT AAGAAAAGGGAGAATGATGGATGTGAAAAAATGTGGCATCCAAGA TACAAACTCAAAGAAGCAAAGTGATACACATTTGGAGGAGACGTAA |

## Claims

1. A method for determining an indicator that is indicative of a subject's Th1 immune status, the method comprising: (1) determining a Th1 immune status biomarker profile of a sample from the subject, wherein the Th1 immune status biomarker profile comprises a first biomarker value that is indicative of an amount of a first Th1 immune status biomarker and a second biomarker value that is indicative of an amount of a second Th1 immune status biomarker in the sample, wherein the first and second Th1 immune status biomarkers are biomarkers on dendritic cells (DCs), wherein the first Th1 immune status biomarker is programmed cell death protein 1 ligand 2 (PD-L2) and the second Th1 immune status biomarker is programmed cell death protein 1 ligand 1 (PD-L1); and (2) determining the indicator using the first and second biomarker values, the indicator being indicative of a sample PD-L2:PD-L1 biomarker value ratio that is indicative of the subject's Th1 immune status, wherein the indicator is determined to be indicative of impaired Th1 immunity in the subject if the sample PD-L2:PD-L1 biomarker value ratio is reduced relative to a control PD-L2:PD-L1 biomarker value ratio that correlates with the presence of normal or unimpaired Th1 immunity, wherein the indicator is determined to be indicative of elevated Th1 immunity in the subject if the sample PD-L2:PD-L1 biomarker value ratio is increased relative to a control PD-L2:PD-L1 biomarker value ratio that correlates with the presence of normal or unimpaired Th1 immunity, and wherein the indicator is determined to be indicative of normal or unimpaired Th1 immunity in the subject if the sample PD-L2:PD-L1 biomarker value ratio is about the same as a control PD-L2:PD-L1 biomarker value ratio that correlates with the presence of normal or unimpaired Th1 immunity.

2. A method according to claim 1, wherein:
a) a respective biomarker value is indicative of a concentration of a corresponding Th1 immune status biomarker in the sample obtained from the subject; and/or
b) a respective biomarker value includes the abundance of a corresponding Th1 immune status biomarker; and/or
c) a respective biomarker value includes the percentage of DCs in the sample that express a corresponding Th1 immune status biomarker on the cell surface; or
d) wherein the first biomarker value is a measurement of PD-L2 clustering on the cell surface of the DCs.

3. A method according to claim 1 or claim 2, wherein the method further comprises applying a combining function to the biomarker values, optionally wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

4. A method according to any one of claims 1 to 3, wherein the biomarker values are measured using microscopy, flow cytometry, immunoassays, mass spectrometry, sequencing platforms, array and hybridization platforms, or a combination thereof,
optionally wherein the immunoassay is an enzyme-linked immunosorbent assay (ELISA) or a radioimmunoassay (RIA).

5. A method according to any one of claims 1 to 4, wherein the indicator is used for diagnosing the presence or absence of a Th1-related disease.

6. A method according to claim 5, wherein:
a) the indicator is determined as being indicative of impaired Th1 immunity and is used for diagnosing a Th1-related disease associated with a reduced or suppressed Th1 immune response, and further optionally wherein the Th1 related disease is a metastatic cancer or a pathogenic infection; or
b) the indicator is determined as being indicative of impaired Th1 immunity and is used for diagnosing a Th1-related disease associated with an elevated Th1 immune response, optionally wherein the Th1-related disease is an autoimmune disease, further optionally wherein the autoimmune disease is selected from: Alzheimer's disease, ankylosing spondylitis, atherosclerosis, autoimmune-associated infertility, autoimmune encephalomyelitis, autoimmune hemolytic anemia, autoimmune hemophilia, autoimmune hepatitis, autoimmune lymphoproliferative syndrome (ALPS), autoimmune thrombocytopenic purpura, autoimmune uveoretinitis, Barrett's esophagus, chronic fatigue syndrome (CFS/CFIDS/ME), bullous pemphigoid, chronic lyme disease (borreliosis), Crohn's disease, diabetes, depression, fibromyalgia (FM), gastritis, gastroesophageal reflux disease (GERD), glomerulonephritis (e.g., crescentic glomerulonephritis, proliferative glomerulonephritis), Goodpasture's syndrome, Grave's disease, Guillain-Barre syndrome, Hashimoto's thyroiditis, hemolytic anemia, hypertension, hyperthyroidism, hypothyroidism, idiopathic Addison's disease, insulin resistance, irritable bowel syndrome (IBS), interstitial cystitis (IC), kidney stones, Löfgren's syndrome, lupus erythematosus, mixed connective tissue disease, multiple chemical sensitivity (MCS), migraine headache, Morgellon's, multiple sclerosis, myasthenia gravis (MG), osteoarthritis, pemphigus (e.g, pemphigus vulgaris), pernicious anemia, polymyalgia rheumatic, polymyositis prostatitis, psoriasis, psoriatic arthritis, Raynaud's syndrome/phenomenon, reactive arthritis (Reiter syndrome), restless leg syndrome, reflex sympathetic dystrophy (RSD), rheumatoid arthritis, sarcoidosis, scleroderma, sinusitis, seasonal affective disorder (SAD), Sjögren's syndrome, ulcerative colitis, uveitis, and vertigo.

7. A method of monitoring a Th1-related disease progression or regression, the method comprising determining disease progression or regression from a first sample from a subject at a point in time and from a second sample from a subject at a later point in time, on the basis of the indicator determined by a method according to any one of claims 1 to 6.

8. A method for determining an indicator that is capable of distinguishing between metastatic cancer and non-metastatic cancer in a subject, the method comprising: (1) determining a Th1 immune status biomarker profile of a sample from the subject, wherein the Th1 immune status biomarker profile comprises a first biomarker value that is indicative of an amount of a first Th1 immune status biomarker and a second biomarker value that is indicative of an amount of a second Th1 immune status biomarker in the sample, wherein the first and second Th1 immune status biomarkers are biomarkers of dendritic cells (DCs), wherein the first Th1 immune status biomarker is programmed cell death protein 1 ligand 2 (PD-L2) and the second Th1 immune status biomarker is programmed cell death protein 1 ligand 1 (PD-L1); and (2) determining the indicator using the first and second biomarker values, the indicator being indicative of a sample PD-L2:PD-L1 biomarker value ratio, wherein the indicator is determined to be indicative of non-metastatic cancer in the subject if the PD-L2:PD-L1 biomarker value ratio is between around 0.9 and 1.3, and wherein the indicator is determined to be indicative of metastatic cancer in the subject if the PD-L2:PD-L1 biomarker value ratio is between around 0.4 and 0.8.

9. A method for determining an indicator that is capable of distinguishing between metastatic cancer and non-metastatic cancer in a subject, the method comprising: (1) determining a Th1 immune status biomarker profile of a sample from the subject, wherein the Th1 immune status biomarker profile comprises a biomarker value that is indicative of a percentage of dendritic cells (DCs) in the sample that express programmed cell death protein 1 ligand 2 (PD-L2) on the cell surface; and (2) determining the indicator using the biomarker value, wherein the indicator is capable of distinguishing between metastatic cancer and non-metastatic cancer in a subject.

10. A method according to claim 9 wherein detecting a PD-L2 biomarker value of around 60% or greater indicates that the subject has a non-metastatic cancer.

11. A method according to claim 9 wherein detecting a PD-L2 biomarker value of less that about 50% indicates that the subject is suffering from a metastatic cancer.

12. A composition for determining an indicator used in assessing a subject's Th1 immune status, or for diagnosing diseases and/or conditions with an undesirable Th1 immune status, or for monitoring disease progression or regression, or for determining an indicator that is useful for distinguishing between a metastatic cancer and a non-metastatic cancer in a subject, the composition comprising, consisting or consisting essentially of dendritic cells (DCs), a PD-L2 detection agent and a PD-L1 detection agent.

13. A composition according to claim 12, wherein:
a) the PD-L2 detection agent and/or PD-L1 detection agent are bound to PD-L2 and/or PD-L1, respectively, on the surface of the DCs; and/or
b) the surface PD-L2 and/or PD-L1 is/are in the form of clusters; and/or
c) the PD-L2 and/or PD-L1 detection agents are antibodies; and/or
d) the PD-L2 and/or PD-L1 detection agents comprise a covalently attached label.

## Patentansprüche

1. Verfahren zum Bestimmen eines Indikators, der auf den Th1-Immunstatus eines Subjekts hinweist, wobei das Verfahren umfasst: (1) Bestimmen eines Th1-Immunstatus-Biomarkerprofils einer von dem Subjekt erhaltenen Probe, wobei das Th1-Immunstatus-Biomarkerprofil einen ersten Biomarkerwert umfasst, der auf eine Menge eines ersten Th1-Immunstatus-Biomarkers hinweist, und einen zweiten Biomarkerwert, der auf eine Menge eines zweiten Th1-Immunstatus-Biomarkers in der Probe hinweist, wobei die ersten und zweiten Th1-Immunstatus-Biomarker Biomarker auf dendritischen Zellen (DCs) sind, wobei der erste Th1-Immunstatus-Biomarker der programmierte Zelltodprotein-1-Ligand-2 (PD-L2) und der zweite Th1-Immunstatus-Biomarker der programmierte Zelltodprotein-1-Ligand 1 (PD-L1) ist; und (2) Bestimmen des Indikators unter Verwendung der ersten und zweiten Biomarkerwerte, wobei der Indikator ein Proben-PD-L2:PD-L1-Biomarkerwert-Verhältnis angibt, das auf den Th1-Immunstatus des Patienten hinweist, wobei bestimmt wird, dass der Indikator eine gestörte Th1-Immunität in dem Subjekt anzeigt, wenn das Proben-PD-L2:PD-L1-Biomarkerwert-Verhältnis relativ zu einem Kontroll-PD-L2:PD-L1-Biomarkerwert-Verhältnis, das mit dem Vorhandensein einer normalen oder ungestörten Th1-Immunität korreliert, reduziert ist, wobei bestimmt wird, dass der Indikator eine erhöhte Th1-Immunität in dem Subjekt anzeigt, wenn das Proben-PD-L2:PD-L1-Biomarker-Wert-Verhältnis relativ zu einem Kontroll-PD-L2:PD-L1-Biomarker-Wert-Verhältnis, das mit dem Vorhandensein einer normalen oder ungestörten Th1-Immunität korreliert, erhöht ist, und wobei bestimmt wird, dass der Indikator eine normale oder ungestörte Th1-Immunität in dem Subjekt anzeigt, wenn das Proben-PD-L2:PD-L1-Biomarker-Wert-Verhältnis etwa gleich ist wie ein Kontroll-PD-L2:PD-L1-Biomarker-Wert-Verhältnis, das mit dem Vorhandensein einer normalen oder ungestörten Th1-Immunität korreliert.

2. Verfahren nach Anspruch 1, wobei:
a) ein jeweiliger Biomarkerwert eine Konzentration eines entsprechenden Th1-Immunstatus-Biomarkers in der von dem Subjekt erhaltenen Probe anzeigt; und/oder
b) ein jeweiliger Biomarkerwert die Häufigkeit eines entsprechenden Th1-Immunstatus-Biomarkers enthält; und/oder
c) ein jeweiliger Biomarkerwert den Prozentsatz der DCs in der Probe enthält, die einen entsprechenden Th1-Immunstatus-Biomarker auf der Zelloberfläche exprimieren; oder
d) wobei der erste Biomarkerwert eine Messung der PD-L2-Anhäufung auf der Zelloberfläche der DCs ist.

3. Verfahren nach Anspruch 1 oder Anspruch 2, wobei das Verfahren ferner die Anwendung einer Kombinationsfunktion auf die Biomarkerwerte umfasst, wobei die Kombinationsfunktion optional mindestens eine der Folgenden ist: ein additives Modell; ein lineares Modell; eine Support-Vektor-Maschine; ein neuronales Netzwerkmodell; ein Random-Forest-Modell; ein Regressionsmodell; ein genetischer Algorithmus; ein Annealing-Algorithmus; eine gewichtete Summe; ein Nearest-Neighbour-Modell; und ein probabilistisches Modell.

4. Verfahren nach einem der Ansprüche 1 bis 3, wobei die Biomarkerwerte unter Verwendung von Mikroskopie, Durchflusszytometrie, Immunoassays, Massenspektrometrie, Sequenzierungsplattformen, Array- und Hybridisierungsplattformen oder einer Kombination davon gemessen werden,
wobei es sich bei dem Immunoassay optional um einen Enzymimmunoassay (ELISA) oder einen Radioimmunoassay (RIA) handelt.

5. Verfahren nach einem der Ansprüche 1 bis 4, wobei der Indikator zur Diagnose des Vorhandenseins oder Nichtvorhandenseins einer Th1-bezogenen Krankheit verwendet wird.

6. Verfahren nach Anspruch 5, wobei:
a) der Indikator als Indikator für eine gestörte Th1-Immunität bestimmt wird und zur Diagnose einer Th1-bezogenen Krankheit verwendet wird, die einer reduzierten oder unterdrückten Th1-Immunantwort zugeordnet ist, und ferner optional, wobei die Th1-bezogene Krankheit ein metastasierender Krebs oder eine pathogene Infektion ist; oder
b) der Indikator als Indikator für eine gestörte Th1-Immunität bestimmt wird und zur Diagnose einer Th1-bezogenen Krankheit verwendet wird, die einer erhöhten Th1-Immunantwort zugeordnet ist, wobei es sich bei der Th1-bezogenen Krankheit optional um eine Autoimmunerkrankung handelt, wobei die Autoimmunerkrankung optional ausgewählt ist aus: Alzheimer-Krankheit, Spondylitis ankylosans, Atherosklerose, Autoimmun-assoziierter Unfruchtbarkeit, Autoimmun-Enzephalomyelitis, Autoimmun-hämolytischer Anämie, Autoimmun-Hämophilie, Autoimmun-Hepatitis, autoimmunem lymphoproliferativem Syndrom (ALPS), autoimmuner thrombozytopenischer Purpura, autoimmuner Uveoretinitis, Barrett-Ösophagus, chronischem Müdigkeitssyndrom (CFS/CFIDS/ME), bullösem Pemphigoid, chronischer Borreliose, Morbus Crohn, Diabetes, Depression, Fibromyalgie (FM), Gastritis, gastroösophagealer Refluxkrankheit (GERD), Glomerulonephritis (z.B. sichelförmiger Glomerulonephritis, proliferativer Glomerulonephritis), Goodpasture-Syndrom, Morbus Grave, Guillain-Barre-Syndrom, Hashimoto-Thyreoiditis, hämolytischer Anämie, Bluthochdruck, Hyperthyreose, Hypothyreose, idiopathischer Addison-Krankheit, Insulinresistenz, Reizdarmsyndrom (IBS), interstitieller Zystitis (IC), Nierensteinen, Lofgren-Syndrom, Lupus erythematodes, gemischter Bindegewebserkrankung, multipler chemischer Sensitivität (MCS), Migränekopfschmerz, Morgellon's, Multipler Sklerose, Myasthenia gravis (MG), Osteoarthritis, Pemphigus (z.B. Pemphigus vulgaris), perniziöser Anämie, Polymyalgia rheumatica, Polymyositis prostatitis, Psoriasis, Psoriasis-Arthritis, Raynaud-Syndrom/Phänomen, reaktiver Arthritis (Reiter-Syndrom), Restless-Leg-Syndrom, Reflex-Sympathikus-Dystrophie (RSD), rheumatoider Arthritis, Sarkoidose, Sklerodermie, Sinusitis, saisonaler affektiver Störung (SAD), Sjogren-Syndrom, Colitis ulcerosa, Uveitis und Schwindel.

7. Verfahren zur Überwachung einer Th1-bezogenen Krankheitsprogression oder -regression, wobei das Verfahren die Bestimmung der Krankheitsprogression oder -regression aus einer ersten Probe, die von einem Subjekt zu einem bestimmten Zeitpunkt erhalten wurde, und aus einer zweiten Probe von einem Subjekt zu einem späteren Zeitpunkt basierend auf dem Indikator umfasst, der durch ein Verfahren nach einem der Ansprüche 1 bis 6 bestimmt wurde.

8. Verfahren zur Bestimmung eines Indikators, der in der Lage ist, zwischen metastasierendem Krebs und nicht-metastasierendem Krebs in einem Subjekt zu unterscheiden, wobei das Verfahren umfasst: (1) Bestimmen eines Th1-Immunstatus-Biomarkerprofils einer von dem Subjekt erhaltenen Probe, wobei das Th1-Immunstatus-Biomarkerprofil einen ersten Biomarkerwert umfasst, der auf eine Menge eines ersten Th1-Immunstatus-Biomarkers hinweist, und einen zweiten Biomarkerwert, der auf eine Menge eines zweiten Th1-Immunstatus-Biomarkers in der Probe hinweist, wobei die ersten und zweiten Th1-Immunstatus-Biomarker Biomarker von dendritischen Zellen (DCs) sind, wobei der erste Th1-Immunstatus-Biomarker der programmierte Zelltodprotein-1-Ligand-2 (PD-L2) und der zweite Th1-Immunstatus-Biomarker der programmierte Zelltodprotein-1-Ligand 1 (PD-L1) ist; und (2) Bestimmen des Indikators unter Verwendung der ersten und zweiten Biomarkerwerte, wobei der Indikator ein Proben-PD-L2:PD-L1-Biomarkerwert-Verhältnis angibt, wobei bestimmt wird, dass der Indikator einen nicht-metastasierenden Krebs in dem Subjekt anzeigt, wenn das PD-L2:PD-L1-Biomarkerwert-Verhältnis zwischen etwa 0,9 und 1,3 liegt, und wobei bestimmt wird, dass der Indikator einen metastasierenden Krebs in dem Subjekt anzeigt, wenn das PD-L2:PD-L1-Biomarkerwert-Verhältnis zwischen etwa 0,4 und 0,8 liegt.

9. Verfahren zur Bestimmung eines Indikators, der in der Lage ist, zwischen metastasierendem Krebs und nicht-metastasierendem Krebs in einem Subjekt zu unterscheiden, wobei das Verfahren umfasst: (1) Bestimmen eines Th1-Immunstatus-Biomarkerprofils einer von dem Subjekt erhaltenen Probe, wobei das Th1-Immunstatus-Biomarkerprofil einen Biomarkerwert umfasst, der einen Prozentsatz von dendritischen Zellen (DCs) in der Probe anzeigt, die den programmierten Zelltodprotein-1-Liganden-2 (PD-L2) auf der Zelloberfläche exprimieren; und (2) Bestimmen des Indikators unter Verwendung des Biomarkerwertes, wobei der Indikator in der Lage ist, zwischen metastasierendem Krebs und nicht-metastasierendem Krebs in einem Subjekt zu unterscheiden.

10. Verfahren nach Anspruch 9, wobei das Erfassen eines PD-L2-Biomarkerwertes von etwa 60% oder mehr anzeigt, dass das Subjekt einen nicht-metastasierenden Krebs hat.

11. Verfahren nach Anspruch 9, wobei das Erfassen eines PD-L2-Biomarkerwerts von weniger als etwa 50% anzeigt, dass das Subjekt an einem metastasierenden Krebs leidet.

12. Zusammensetzung zur Bestimmung eines Indikators, der zur Beurteilung des Th1-Immunstatus eines Subjekts oder zur Diagnose von Krankheiten und/oder Zuständen mit einem unerwünschten Th1-Immunstatus oder zur Überwachung des Fortschreitens oder der Regression einer Krankheit verwendet wird, oder zur Bestimmung eines Indikators, der zur Unterscheidung zwischen einem metastasierten Krebs und einem nicht-metastasierten Krebs in einem Subjekt nützlich ist, wobei die Zusammensetzung dendritische Zellen (DCs), ein PD-L2-Nachweismittel und ein PD-L1-Nachweismittel umfasst, daraus besteht oder im Wesentlichen daraus besteht.

13. Zusammensetzung nach Anspruch 12, wobei:
a) das PD-L2-Nachweismittel und/oder das PD-L1-Nachweismittel an PD-L2 und/oder PD-L1 auf der Oberfläche der DCs gebunden sind; und/oder
b) das Oberflächen-PD-L2 und/oder PD-L1 in Form von Clustern vorliegt; und/oder
c) das PD-L2- und/oder PD-L1-Nachweismittel Antikörper sind; und/oder
d) das PD-L2- und/oder PD-L 1 -Nachweismittel eine kovalent gebundene Markierung umfassen.

## Revendications

1. Procédé pour déterminer un indicateur qui est indicatif d'un état immunitaire Th1 d'un sujet, le procédé comprenant : (1) la détermination d'un profil de biomarqueur d'état immunitaire Th1 d'un échantillon provenant du sujet, dans lequel le profil de biomarqueur d'état immunitaire Th1 comprend une première valeur de biomarqueur qui est indicative d'une quantité d'un premier biomarqueur d'état immunitaire Th1 et une seconde valeur de biomarqueur qui est indicative d'une quantité d'un second biomarqueur d'état immunitaire Th1 dans l'échantillon, dans lequel les premier et second biomarqueurs d'état immunitaire Th1 sont des biomarqueurs sur des cellules dendritiques (CDs), dans lequel le premier biomarqueur d'état immunitaire Th1 est protéine ligand-1 de mort cellulaire programmée 2 (PD-L2) et le second biomarqueur d'état immunitaire Th1 est protéine ligand-1 de mort cellulaire programmée 1 (PD-L1) ; et (2) la détermination de l'indicateur en utilisant les première et seconde valeurs de biomarqueur, l'indicateur étant indicatif d'un rapport de valeurs de biomarqueurs PD-L2 : PD-L1 d'échantillon qui est indicatif de l'état immunitaire Th1 du sujet, dans lequel l'indicateur est déterminé être indicatif d'immunité Th1 déficiente chez le sujet si le rapport de valeurs de biomarqueurs PD-L2 : PD-L1 d'échantillon est réduit relativement à un rapport de valeurs de biomarqueurs PD-L2 : PDL1 de contrôle qui est corrélé avec la présence d'immunité Th1 normale ou déficiente, dans lequel l'indicateur est déterminé être indicatif d'immunité Th1 élevée chez le sujet si le rapport de valeurs de biomarqueurs PD-L2 : PD-L1 d'échantillon est augmenté relativement à un rapport de valeurs de biomarqueurs PD-L2 : PD-L1 de contrôle qui est corrélé avec la présence d'immunité Th1 normale ou déficiente, et dans lequel l'indicateur est déterminé être indicatif d'immunité Th1 normale ou déficiente chez le sujet si le rapport de valeurs de biomarqueurs PD-L2 : PD-L1 d'échantillon est environ le même qu'un rapport de valeurs de biomarqueurs PD-L2 : PD-L1 de contrôle qui est corrélé avec la présence d'immunité Th1 normale ou déficiente.

2. Procédé selon la revendication 1, dans lequel :
a) une valeur de biomarqueur respective est indicative d'une concentration en un biomarqueur d'état immunitaire Th1 correspondant dans l'échantillon obtenu du sujet ; et/ou
b) une valeur de biomarqueur respective inclut l'abondance d'un biomarqueur d'état immunitaire Th1 correspondant ; et/ou
c) une valeur de biomarqueur respective inclut le pourcentage de CDs dans l'échantillon qui expriment un biomarqueur d'état immunitaire Th1 correspondant sur la surface cellulaire ; ou
d) dans lequel la première valeur de biomarqueur est une mesure de PD-L2 se regroupant sur la surface cellulaire des CDs.

3. Procédé selon la revendication 1 ou la revendication 2, dans lequel le procédé comprend en outre
l'application d'une fonction de combinaison sur les valeurs de biomarqueurs, optionnellement dans lequel la fonction de combinaison est au moins un de : un modèle additif ; un modèle linéaire ; une machine à vecteur de support ; un modèle de réseau neuronal ; un modèle de forêt aléatoire ; un modèle de régression ; un algorithme génétique ; un algorithme de recuit ; une somme pondérée ; un modèle de voisins les plus proches ; et un modèle probabiliste.

4. Procédé selon l'une quelconque des revendications 1 à 3, dans lequel les valeurs de biomarqueurs sont mesurées en utilisant une microscopie, une cytométrie en flux, des immunoanalyses, une spectrométrie de masse, des plates-formes de séquençage, plates-formes d'analyse et d'hybridisation, ou une association de celles-ci,
optionnellement dans lequel l'immunoanalyse est un ELISA (« enzyme-linked immunosorbent assay ») ou une radioimmunoanalyse (RIA).

5. Procédé selon l'une quelconque des revendications 1 à 4, dans lequel l'indicateur est utilisé pour diagnostiquer la présence ou l'absence d'une maladie connexe à Th1.

6. Procédé selon la revendication 5, dans lequel :
a) l'indicateur est déterminé comme étant indicatif d'immunité Th1 déficiente et est utilisé pour diagnostiquer une maladie connexe à Th1 associée à une réponse immunitaire Th1 réduite ou déprimée, et en outre optionnellement dans lequel la maladie connexe à Th1 est un cancer métastatique ou une infection pathogène ; ou
b) l'indicateur est déterminé comme étant indicatif d'immunité Th1 déficiente et est utilisé pour diagnostiquer une maladie connexe à Th1 associée à une réponse immunitaire Th1 élevée, optionnellement dans lequel la maladie connexe à Th1 est une maladie auto-immune, en outre optionnellement dans lequel la maladie auto-immune est sélectionnée parmi : maladie d'Alzheimer, spondylite ankylosante, athérosclérose, infertilité associée à l'auto-immunité, encéphalomyélite auto-immune, anémie hémolytique auto-immune, hémophilie auto-immune, hépatite auto-immune, syndrome lymphoprolifératif auto-immun (SLAI), purpura thrombocytopénique auto-immun, uvéo-rétinite auto-immune, oesophage de Barrett, syndrome de fatigue chronique (SFC/SFCDI/EM), pemphigoïde bulleuse, maladie de Lyme chronique (borréliose), maladie de Crohn, diabète, dépression, fibromyalgie (FM), gastrite, maladie de reflux gastro-oesophagien (MRGO), glomérulonéphrite (par exemple, glomérulonéphrite crescentique, glomérulonéphrite proliférative), syndrome de Goodpasture, maladie de Grave, syndrome de Guillain-Barré, thyroïdite de Hashimoto, anémie hémolytique, hypertension, hyperthyroïdisme, hypothyroïdisme, maladie d'Addison idiopathique, résistance à l'insuline, syndrome de l'intestin irritable (SII), cystite interstitielle (CI), calculs rénaux, syndrome de Lofgren, lupus érythémateux, maladie du tissu conjonctif mixte, sensibilité chimique multiple (SCM), migraine, maladie des Morgellon, sclérose en plaques, myasthénie grave (MG), ostéoarthrite, pemphigus (par exemple pemphigus vulgaris), anémie pernicieuse, polymyalgie rhumatoïde, polymyosite prostatite, psoriasis, arthrite psoriasique, syndrome/phénomène de Raynaud, arthrite réactive (syndrome de Reiter), syndrome des jambes sans repos, dystrophie sympathique réflexe (DSR), arthrite rhumatoïde, sarcoïdose, scléroderme, sinusite, trouble affectif saisonnier (TAS), syndrome de Sjogren, colite ulcérative, uvéite, et vertige.

7. Procédé de surveillance d'une progression ou de régression de maladie connexe à Th1, le procédé comprenant la détermination de progression ou de régression de maladie à partir d'un premier échantillon provenant d'un sujet, à un instant, et à partir d'un second échantillon provenant d'un sujet, à un instant ultérieur, sur la base de l'indicateur déterminé par un procédé selon l'une quelconque des revendications 1 à 6.

8. Procédé pour déterminer un indicateur qui est capable de distinguer entre un cancer métastatique et un cancer non métastatique chez un sujet, le procédé comprenant : (1) la détermination d'un profil de biomarqueur d'état immunitaire Th1 d'un échantillon provenant du sujet, dans lequel le profil de biomarqueur d'état immunitaire Th1 comprend une première valeur de biomarqueur qui est indicative d'une quantité d'un premier biomarqueur d'état immunitaire Th1 et une seconde valeur de biomarqueur qui est indicative d'une quantité d'un second biomarqueur d'état immunitaire Th1 dans l'échantillon, dans lequel les premier et second biomarqueurs d'état immunitaire Th1 sont des biomarqueurs de cellules dendritiques (CDs), dans lequel le premier biomarqueur d'état immunitaire Th1 est protéine ligand-1 de mort cellulaire programmée 2 (PD-L2) et le second biomarqueur d'état immunitaire Th1 est protéine ligand-1 de mort cellulaire programmée 1 (PD-L1) ; et (2) la détermination de l'indicateur en utilisant les première et seconde valeurs de biomarqueur, l'indicateur étant indicatif d'un rapport de valeurs de biomarqueurs PD-L2 : PD-L1 d'échantillon, dans lequel l'indicateur est déterminé être indicatif de cancer non métastatique chez le sujet si le rapport de valeurs de biomarqueurs PD-L2 : PD-L1 est entre environ 0,9 et 1,3, et dans lequel l'indicateur est déterminé être indicatif de cancer métastatique chez le sujet si le rapport de valeurs de biomarqueurs PD-L2 : PD-L1 est entre environ 0,4 et 0,8.

9. Procédé pour déterminer un indicateur qui est capable de distinguer entre un cancer métastatique et un cancer non métastatique chez un sujet, le procédé comprenant : (1) la détermination d'un profil de biomarqueur d'état immunitaire Th1 d'un échantillon provenant du sujet, dans lequel le profil de biomarqueur d'état immunitaire Th1 comprend une valeur de biomarqueur qui est indicative d'un pourcentage de cellules dendritiques (CDs) dans l'échantillon qui expriment une protéine ligand-1 de mort cellulaire programmée 2 (PD-L2) sur la surface cellulaire ; et (2) la détermination de l'indicateur en utilisant la valeur de biomarqueur, dans lequel l'indicateur est capable de distinguer entre un cancer métastatique et un cancer non métastatique chez un sujet.

10. Procédé selon la revendication 9, dans lequel la détection d'une valeur de biomarqueur PD-L2 d'environ 60 % ou plus indique que le sujet a un cancer non métastatique.

11. Procédé selon la revendication 9, dans lequel la détection d'une valeur de biomarqueur PD-L2 de moins d'environ 50 % indique que le sujet est atteint d'un cancer métastatique.

12. Composition pour déterminer un indicateur utilisé dans l'évaluation d'un état immunitaire Th1 de sujet, ou pour diagnostiquer des maladies et/ou conditions avec un état immun Th1 indésirable, ou pour surveiller une progression ou régression de maladie, ou pour déterminer un indicateur qui est utile pour distinguer entre un cancer métastatique et un cancer non métastatique chez un sujet, la composition comprenant des cellules dendritiques (CDs), un agent de détection de PD-L2 et un agent de détection de PD-L1, ou étant constituée ou étant essentiellement constituée de ceux-ci.

13. Composition selon la revendication 12, dans lequel :
a) l'agent de détection de PD-L2 et/ou l'agent de détection de PD-L1 sont liés à PD-L2 et/ou PD-L1, respectivement, sur la surface des CDs ; et/ou
b) la surface PD-L2 et/ou PD-L1 est/sont sous la forme de groupements ; et/ou
c) les agents de détection de PD-L2 et/ou de PD-L1 sont des anticorps ; et/ou
d) les agents de détection de PD-L2 et/ou PD-L1 comprennent une étiquette attachée de façon covalente.
